**Europäisches Patentamt**

**European Patent Office**

**Office européen des brevets**

(19)

(11) **EP 1 170 011 A1**

(12) **EUROPEAN PATENT APPLICATION**

(43) Date of publication:
**09.01.2002 Bulletin 2002/02**

(51) Int Cl.[7]: **A61K 31/5377**, A61K 31/519,
A61K 31/517, A61P 35/00

(21) Application number: **00114482.3**

(22) Date of filing: **06.07.2000**

| | |
|---|---|
| (84) Designated Contracting States:<br>**AT BE CH CY DE DK ES FI FR GB GR IE IT LI LU MC NL PT SE**<br>Designated Extension States:<br>**AL LT LV MK RO SI** | • **Wagner, Erwin F., Prof.Dr.**<br>**1030 Wien (AT)**<br>• **Behrens, Axel, Dr.**<br>**8005 Zürich (CH)**<br>• **Fleischmann, Alexander**<br>**1030 Wien (AT)**<br>• **Metz, Thomas, Dr.**<br>**1020 Wien (AT)** |
| (71) Applicant: **Boehringer Ingelheim International GmbH**<br>**55218 Ingelheim am Rhein (DE)** | |
| (72) Inventors:<br>• **Sibilia, Maria, Dr.**<br>**1030 Wien (AT)** | (74) Representative: **Laudien, Dieter, Dr. et al**<br>**Boehringer Ingelheim International GmbH ZA**<br>**Patente Postfach 200**<br>**55216 Ingelheim am Rhein (DE)** |

(54) **Novel use of inhibitors of the epidermal growth factor receptor**

(57)      Epidermal growth factor receptor (EGFR) inhibitors for the preparation of a medicament for the treatment of tumors whose cells proliferate as a result of a deregulated mitogenic signal transduction pathway and require the function of wildtype EGFR as a survival factor and as an inhibitor of differentiation.

Fig. 6b

**Description**

The present invention relates to the therapy of cancer.

The epidermis is a stratified squamous epithelium composed mainly of keratinocytes, whose proliferation and differentiation must be tightly regulated and coordinated (Fuchs, 1990; Fuchs, 1992). Basal keratinocytes, which are attached to the basement membrane, are undifferentiated and have proliferative potential. Before entering the differentiation program, they withdraw from the cell cycle and migrate towards the surface of the epidermis leading to the formation of the outermost layer of the epidermis composed of anucleated dead squames, which are continuously shed from the surface of the skin (Jones and Watt, 1993).

Epidermal growth factor receptor (EGFR) activation is a central event in the regulation of epidermal development. The EGFR is activated by several ligands such as epidermal growth factor (EGF), transforming growth factor $\alpha$ (TGF$\alpha$), amphiregulin, heparin-binding EGF (HB-EGF), betacellulin and epiregulin (Earp et al., 1995; Prigent and Lemoine, 1992). Ligand binding to the EGFR induces receptor dimerization and activation of the intrinsic tyrosine kinase with subsequent autophosphorylation of key tyrosines located at the carboxyl terminal tail of the receptor (Earp et al., 1995; Lemmon and Schlessinger, 1994; Prigent and Lemoine, 1992). Phosphorylated tyrosine residues act as binding sites for proteins containing Src-homology 2 domains (SH2) such as Grb2, SHC and PLC$\gamma$ which, in turn, activate complex downstream signaling cascades thus transducing extracellular stimuli to the nucleus (Lemmon and Schlessinger, 1994; Weiss et al., 1997). The adapter protein Grb2 seems to be critically involved in coupling signals from receptor tyrosine kinases to Ras through its association with Son of sevenless (SOS), a guanine nucleotide exchange factor that catalyzes the activation of Ras proteins by facilitating GDP-GTP exchange (Schlessinger, 1994; Weiss et al., 1997). Stimulation of cells with growth factors leads to the association of SOS-Grb2 complexes with activated receptors, and this is proposed to stimulate Ras through the juxtaposition of SOS and Ras at the membrane (Schlessinger, 1994; Weiss et al., 1997). Constitutive active SOS proteins can be obtained by targeting variants of SOS to the membrane via the addition of farnesylation signals (Aronheim et al., 1994). Moreover, deletion of the carboxyl terminal tail of SOS containing the Grb2 binding site also activates the SOS protein (Wang et al., 1995). These dominant forms of SOS have been shown to transform NIH3T3 fibroblasts *in vitro* and to constitutively activate the Ras/ERK pathway (Aronheim et al., 1994; Wang et al., 1995).

Numerous studies have documented alterations in growth factor signaling pathways in the development of human epithelial neoplasms (Derynck, 1992; Reichmann, 1994). Amplifications, rearrangements and overexpression of the EGFR have been shown to occur at high frequency in human squamous cell carcinomas and glioblastomas (Derynck, 1992; Libermann et al., 1985) and activating mutations of the *ras* gene are observed in a variety of human neoplasms (Barbacid, 1990). Introduction of viral Ras into mouse epidermal cells *in vivo* and *in vitro* can initiate skin tumors (Brown et al., 1986; Roop et al., 1986) and expression of an activated form of Ha-Ras in the suprabasal layer of the epidermis of transgenic mice induces the development of benign papillomas at sites of promotional stimuli (Bailleul et al., 1990). Similarly, transgenic mice expressing the EGFR ligand TGF$\alpha$ in basal or suprabasal keratinocytes display thickening of the epidermis and develop papillomas prevalently at sites of mechanical irritations or wounding (Dominey et al., 1993; Vassar and Fuchs, 1991). In contrast, transgenic mice expressing an activated Ras in the outer root sheath of the hair follicles develop spontaneous papilloma-like skin tumors that frequently undergo conversion to squamous carcinomas (Brown et al., 1998).

EGFR signaling is also of physiological relevance during normal epithelial development. Some of the EGFR ligands are synthesized by normal keratinocytes both *in vitro* and *in vivo* (Vardy et al., 1995). The EGFR is most strongly expressed in the basal layer of the epidermis and in the outer root sheath of hair follicles, in which the proliferating keratinocytes reside (King et al., 1990; Sibilia and Wagner, 1995). The number of receptors decreases as keratinocytes migrate to the suprabasal layers of the epidermis, entering the pathway of terminal differentiation (King et al., 1990). Recently, it was shown that EGFR signaling regulates keratinocyte survival, since antibody-mediated inhibition of the EGFR renders keratinocytes detached from the extracellular matrix susceptible to apoptosis (Rodeck et al., 1997a).

Mice deficient for the TGF$\alpha$ gene develop a wavy coat and curly whiskers (Luetteke et al., 1993; Mann et al., 1993). A similar phenotype is observed in the naturally occurring mouse mutant strain waved-2 (wa2), which is homozygous for a hypomorphic EGFR allele. Wa2 mice carry a point mutation in the kinase domain of the EGFR resulting in reduced kinase activity (Fowler et al., 1995; Luetteke et al., 1994). In contrast, mice harboring a null mutation in the EGFR gene exhibit strain-dependent phenotypes with defects in neural and epithelial tissues and die before weaning age (Miettinen et al., 1995; Sibilia et al., 1998; Sibilia and Wagner, 1995; Threadgill et al., 1995). These mutants show impaired epidermal as well as hair follicle differentiation and fail to develop a hairy coat most likely because EGFR signaling is necessary for maintenance of hair follicle integrity (Hansen et al., 1997; Miettinen et al., 1995; Sibilia and Wagner, 1995; Threadgill et al., 1995). Similar skin and hair phenotypes are observed in transgenic mice expressing a dominant negative EGFR (CD533) in the basal layer of the epidermis and outer root sheath of the hair follicles (Murillas et al., 1995). These results suggest that TGF$\alpha$/EGFR signaling plays an essential role in epithelial cell proliferation and/or differentiation and is critical for the development of normal hair follicles and skin. However, it is unclear which signaling pathways downstream of the EGFR specifically control these processes.

Since deregulation of EGFR function has been implicated in various diseases, in particular various types of cancer, the EGFR was suggested as a target molecule for the therapy of cancers. Numerous EGFR inhibitors have been described for the therapy of cancers which are caused by excess EGFR function, in particular EGFR amplification, overexpression or mutations which make the EGFR constitutively active (for review, see, for example, Modjtahedi and Dean, 1994)

It was an object of the invention to investigate EGFR responsive pathways in order to harness the findings of this investigation for a novel therapy for certain cancers.

To this end, mouse skin epithelium was used as a model. Transgenic mice expressing an activated form of hSOS (SOS-F) under the control of a full-length K5 promoter, which is active in EGFR-expressing cells have been generated. It could be shown that all K5-SOS-F mice develop spontaneous skin tumors and that tumor development is impaired if EGFR function is reduced, likely due an increase of apoptosis and differentiation. This demonstrates that the EGFR functions as a survival factor and a differentiation inhibitor in oncogenic transformation caused by a deregulation of the Ras signal transduction pathway.

In the experiments of the present invention, it has been shown that K5-SOS-F transgenic mice develop spontaneous skin tumors, which resemble the lesions observed in transgenic mice expressing an activated Ras in hair follicles (Brown et al., 1998) suggesting that hyperkeratosis may result from activation of the Ras signaling pathway by SOS. Epidermal thickening and papilloma formation were also observed in K14-TGFα transgenic mice. However, the epidermal hyperplasia of K14-TGFα mice regressed with time and terminal differentiation was not perturbed (Vassar and Fuchs, 1991). As EGFR expression is unaffected in K14-TGFα mice, it seems that ligand-induced EGFR stimulation alone is not capable of inducing epidermal hyperproliferation and that constitutive activation of the EGFR or SOS/Ras are required to trigger tumor formation (Vassar and Fuchs, 1991).

Ras activation has been shown to directly affect terminal differentiation of keratinocytes. Retroviral infection of v-*ras* in wild-type keratinocytes results in suppression of K1 and K10 expression and Protein Kinase C (PKC) activity, which is thought to promote keratinocyte differentiation (Denning et al., 1993; Dlugosz et al., 1994). In contrast, v-*ras* infected EGFR-/-keratinocytes do not downregulate K1 and K10 and maintain high PKC activity indicating that EGFR might negatively regulate keratinocyte terminal differentiation (Denning et al., 1996; Dlugosz et al., 1997). A higher number of K1/10 positive keratinocytes was detected in wa2/- K5-SOS-F keratinocytes and papillomas supporting the hypothesis that impaired EGFR signaling promotes keratinocyte differentiation. The premature differentiation might in part explain why papilloma development is severely impaired in a EGFR null or wa2/wa2 background. In K5-SOS-F papillomas as well as in primary keratinocytes the number of proliferating cells was increased to the same extent in wild-type and wa2/wa2 background. Similarly, the EGFR does not seem to be required in v-*ras* mediated keratinocyte proliferation, since the proliferation capacity of v-*ras* infected EGFR-/- or +/+ keratinocytes was comparable (Dlugosz et al., 1997). These results suggest that a functional EGFR is not required for proliferation of SOS-F or Ras transformed cells, but that EGFR signaling might negatively affect terminal differentiation of basal keratinocytes.

In the absence of a wild-type EGFR, K5-SOS-F papillomas and keratinocytes show increased apoptosis suggesting that the EGFR also provides a survival signal for tumor cells. It was shown that squamous cell papillomas produced by grafting v-*ras* infected EGFR-/- keratinocytes onto nude mice were smaller than EGFR wild-type Ras tumors although no significant increase in apoptosis was detected (Dlugosz et al., 1997). It is possible that the high expression levels of constitutively active viral Ras results in maximal stimulation of direct downstream targets such as PI3-kinase, which mediates cell survival via Akt, so that an additional survival input from the EGFR is functionally insignificant. In K5-SOS-F keratinocytes Ras, which is the direct target of SOS, would still be a limiting factor in the signaling cascade thus avoiding to activate multiple effector pathways. Therefore, an additional SOS/Ras-independent survival signal originating from the EGFR becomes essential for tumor development.

The EGFR has been implicated in the regulation of cell survival in epithelial cells. EGFR activation in keratinocytes can lead to transcription of bcl-X$_L$, a member of the bcl-2 family of proteins and human keratinocytes show increased apoptosis when treated *in vitro* with blocking EGFR antibodies or EGFR-specific tyrosine kinase inhibitors (Rodeck et al., 1997a; Rodeck et al., 1997b; Stoll et al., 1998). However, little is known about the signal transduction pathways linking the EGFR to regulation of cell survival. Oncogenic Ras has been shown to regulate both pro- and anti-apoptotic pathways (Downward, 1998). Cell survival is mediated by P13-kinase and Akt whereas the Raf pathway is required for Ras-induced apoptosis (Downward, 1998). In wa2/- keratinocytes ERK1/2 activation, which is directly regulated by Raf, is not markedly altered even in the presence of K5-SOS-F. In contrast, Akt phosphorylation is reduced in wa2/- K5-SOS-F keratinocytes indicating that an EGFR-dependent signal is necessary for maximal Akt activation. Although it is tempting to speculate that Akt is a mediator of the survival function of the EGFR, it can not be excluded that there are additional molecular targets of EGFR-dependent survival signaling. Recently it was shown in the fruit fly *Drosophila melanogaster* that EGFR signaling via the Ras/ERK pathway promotes cell survival by downregulating the expression of the pro-apoptotic gene *hid* (Bergmann et al., 1998; Kurada and White, 1998) suggesting that two independent pathways, one by Ras/ERK and one by the EGFR have to converge for efficient repression of such pro-apoptotic signals. The survival function of the EGFR is not only restricted to SOS-F-transformed keratinocytes but can also be demonstrated in mesenchymal cells, since EGFR-/- fibroblasts and NIH 3T3 cells expressing a dominant negative EGFR are also resistant to transformation by oncogenic forms of hSOS and Ras. Interestingly, expression of the anti-apoptotic gene *bcl*-2 in SOS-F-transfected EGFR-/-fibroblasts restores transformation and the capability to form tumors in nude mice.

Overexpression of members of the Ras signaling pathway have been shown to induce the secretion of EGFR ligands and therefore activate an autocrine loop contributing to cellular transformation (Gangarosa et al., 1997; McCarthy et al., 1995). An increase in the expression of the EGFR ligand HB-EGF was observed in transgenic keratinocytes in both wa2/+ and wa2/- backgrounds. Therefore, the increased HB-EGF expression in the presence of K5-SOS-F might establish an autocrine stimulation of the EGFR that contributes to tumor development. However, since the EGFR gets activated in both, wa2/+ and wa2/- transgenic keratinocytes, this alone is unlikely to account for the differences in tumor formation. Reduced tumor development in an EGFR hypomorphic background is most likely not caused by the lack of a wild-type receptor in the dermis, since wa2/+ K5-SOS-F keratinocytes grafted in combination with wa2/- dermal fibroblasts onto nude mice form papillomas that are similar to the ones developing in a wild-type EGFR background.

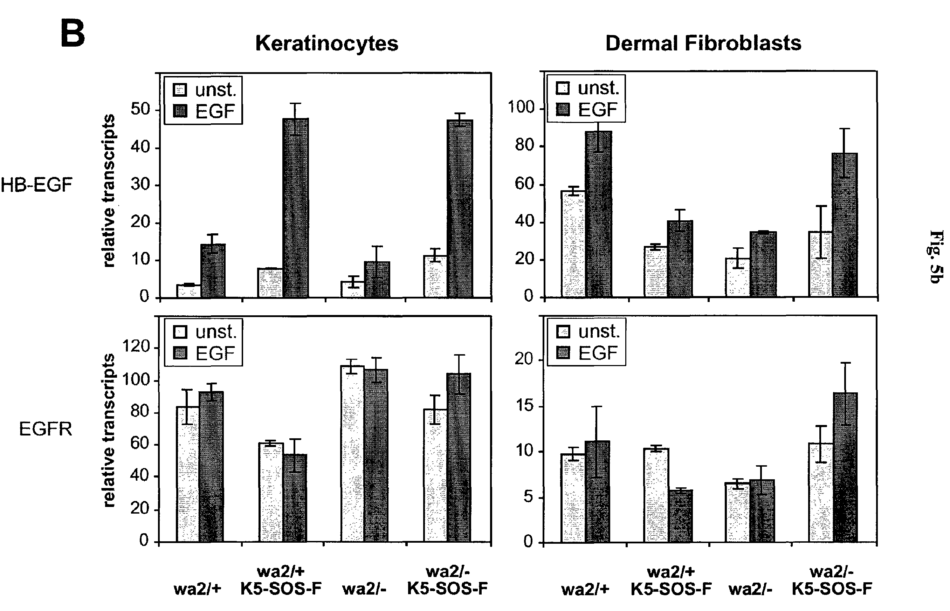

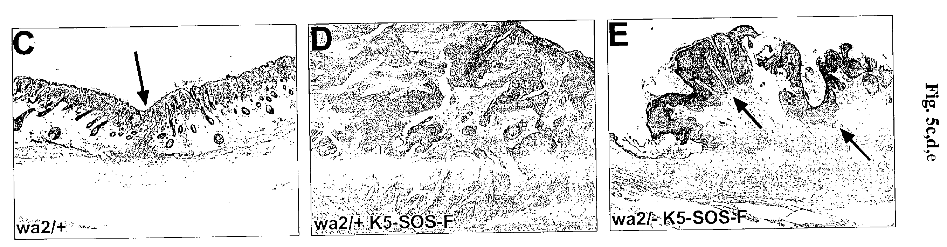

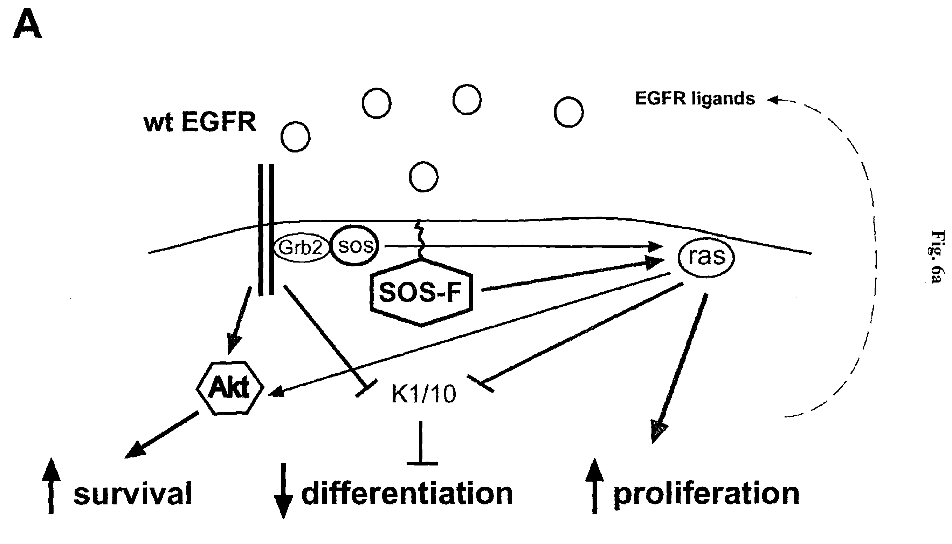

The findings of the experiments of the present invention have revealed that EGFR signaling appears to have two functions during skin development: it provides a survival signal by activating an anti-apoptotic pathway and inhibits keratinocyte differentiation thereby keeping basal cells in the proliferative compartment attached to the basal membrane. K5-SOS-F keratinocytes are prone to hyperproliferation independent of whether a wild-type or mutant EGFR is present. In a wild-type EGFR background, hyperproliferation induced by SOS-F together with the survival signal provided by the EGFR will lead to skin tumor development (Fig. 6A). In a wa2/- transgenic background, SOS-F expression will still lead to hyperproliferation, however, keratinocytes will be induced to leave the basal compartment prematurely, since the absence of the EGFR favors terminal differentiation. Moreover, the survival signal activated by the EGFR possibly by Akt is missing and due to these conflicting signals, keratinocytes would be committed to apoptose (Kauffmann-Zeh et al., 1997). As a consequence no tumors develop in the absence of a functional EGFR (Fig. 6B). Since *in vitro* keratinocytes are grown on plastic substrates, this might also explain why undifferentiated SOS transgenic keratinocytes in a wa2/wa2 background do not show increased apoptosis unless they are induced to differentiate by placing them in suspension.

A similar scenario could be true for other human tumors, which carry activating mutations in components of the Ras signaling pathway. The tumor cells would be prone to hyperproliferation and the EGFR (or other growth factor receptors) may additionally provide survival signals. The EGFR itself is frequently amplified, overexpressed or rearranged in human squamous cell carcinomas and glioblastomas (Derynck, 1992; Libermann et al., 1985). Thus far, it has never been clarified whether the EGFR simply provides a proliferation advantage to tumor cells or if other cellular processes are altered. Since EGFR amplifications and rearrangements often occur at later stages of tumor development, one would exclude that the only function of the EGFR is to provide a proliferation advantage. It is likely that the EGFR triggers multiple downstream survival pathways, rendering tumor cells more aggressive and resistant to chemotherapeutic drugs which usually induce apoptosis by activating only one of the apoptotic programs (Nagane et al., 1998).

The present invention is based on the surprising finding that the EGFR has the potential to function as a potent survival factor and as an inhibitor of the differentiation of tumor cells whose proliferation is driven by an activated mitogenic signal transduction pathway.

Therefore, the present invention relates to EGFR inhibitors for the preparation of a medicament for the treatment of tumors whose cells proliferate as a result of a deregulated mitogenic signal transduction pathway and require the function of wildtype EGFR as a survival factor and as an inhibitor of differentiation.

Within the meaning of the present invention, the term "deregulated mitogenic pathway" encompasses any excess activity in signal transduction resulting from an overexpression or structural change, e.g. due to a point mutation or deletion, of any protein component in the Ras signal transduction pathway.

In a preferred embodiment, the EGFR inhibitors are used for the therapy of tumors in which an excess activity of a component of the Ras signal transduction pathway is involved.

Representatives of components of the Ras signal transduction pathway are SOS, Ras itself, Raf, MEK, and Erk.

Since the EGFR may also be involved as a survival factor and differentiation inhibitor in tumors, in which the mitogenic stimulus is provided by the excess activity of intracellular signal transducers outside or upstream of the Ras pathway such as Grb2, Shc, Abl and Src, EGFR inibitors may also be useful for the inhibition of cancer caused by such excess activity.

"Excess activity" is defined as an activity which originates from overexpression and/or activating mutations of the respective component of the signal transduction pathway and results in a constitutive mitogenic signal even in the absence of stimulating growth factors.

To date, EGFR inhibitors have been suggested for the treatment of malignancies which are driven by a deregulated EGFR, as defined above. According to the current view, the EGFR inhibitors are effective in the treatment of cancer only in the case that they interfere with deregulated EGFR function. In contrast to this view, according to the present invention EGFR inhibitors can be successfully employed for the treatment of tumors due to their ability to inhibit the normal wildtype EGFR function as a survival factor and as an inhibitor of the differentiation of tumor cells, the proliferation of which is driven by a deregulated mitogenic pathway, which remains unaffected upon application of the EGFR inhibitor.

Deregulation of mitogenic pathways, e.g. the Ras pathway, has been observed in many different tumor types, e.g. colon cancer, pancreatic cancer and cancer of the lung (Kiaris and Spandidos, 1995). Mutations of Ras itself have been implicated in approximately 30% of all human tumors. Most of these tumors have a bad prognosis, in part because no effective Ras inhibitors have become available to date. The present invention provides a novel therapeutic approach to treat these tumors and thus meets a so far unmet medical need.

For use in the present invention, any compound is suitable which inhibits binding of activating EGFR ligands, either by binding to the ligand or to EGFR. Examples for the latter are inhibitory anti-EGFR monoclonal antibodies which act as receptor antagonists and have been described by Modjtahedi et al.,.1998; Fong et al., 1992; Prevett et al., 1996; Normanno et al., 1999.

Alternatively, the EGFR inhibitors useful in the present invention are antisense oligonucleotides inhibiting expression of the EGFR gene or of a gene encoding one of the EGFR ligands. An example of such an antisense inhibitor was described by Normanno et al., 1999.

Most preferred are low molecular weight chemical compounds which act as inhibitors of the EGFR tyrosine kinase activity.

Examples of EGFR low molecular weight inhibitors of this type have been described by Strawn and Shawver, 1998 Expert Opinion On Investigational Drugs 1998, 7 (4), 553 -573),and McMahon et al, 1998 inhibitors are also described in WO 96/07657, WO 97/01047, WO 97/01058, WO 97/01057, WO 96/33980, e.g. N-(3-chloro-4-fluorophenyl)-7-methoxy-6-[3-(4-morpholinyl)propoxy]-4-chinazolinamine (ZD-1839); WO 96/30347, e.g. N-(3-ethinylphenyl)-6,7-bis(2-methoxyethoxy)-4-chinazolinamine (CP 358774); WO 97/38983, e.g. N-(4-(3-(chloro-4-fluoro-phenylamino-7-(3-morpholine-4-yl-propoxy)-chinazoline-6-yl)-acrylamiddihydrochloride (Cl 1033, PD 183805); WO 97/02266 (phenylaminopyrrolopyrimidine; PKI-166); examples of protein kinase inhibitors are also given in Current Opinion in Drug Discovery & Development 1998 1 (2): 131-146.

In an experiment of the present invention, it has been shown that the growth of nude mouse xenografts of a human colon adenocarcinoma which expresses normal levels of wildtype EGFR and carries an activated Ras gene is inhibited by an inhibitor of the EGFR tyrosine kinase activity.

Within the scope of the present invention, EGFR inhibitors are applied to tumor patients whose tumor exhibits excess activity of a component of a mitogenic signal transduction pathway, in particular the Ras pathway. In this case, the EGFR inhibitors are applied to patients with Ras mutations. Ras mutations, in particular the mutations at codons 12, 13 and 61 of the K-ras oncogene, which have been implicated in various malignancies (Kiaris et al., 1995; Bos 1988, Mutation Res. 195: 255-271), can be identified routinely, e.g. by means of PCR-based methods (e.g. Roberts et al., 1999). Such methods, which are commercially availabe for diagnostic purposes, can be used for the analysis of tumor biopsies or for non-invasive testing of samples from the patient, e.g., in the case of colon cancer, by detecting the mutation in feces (Hasegawa et al., 1995), or, in the case of lung cancer, by analysis of bronchoalveolar lavage (Lehman et al., 1996).

Within the scope of the invention, the EGFR inhibitors may be used on their own or in conjunction with other pharmacologically active compounds, in particular with other anti-tumor therapeutic agents, for example in combination with topoisomerase inhibitors (e.g. etoposide), mitosis inhibitors (e.g. vinblastin), compounds which interact with nucleic acids (e.g. cis-platin, cyclophosphamide, adriamycin), hormone antagonists (e.g. tamoxifen), inhibitors of metabolic processes (e.g. 5-FU, gemcitabine etc.), cytokines (e.g. interferons), antibodies, etc. For treating respiratory tract tumors, the EGFR inhibitors, optionally in combination with other antitumor drugs, may be used on their own or in conjunction with other therapeutic agents for the airways, such as substances with a secretolytic, broncholytic and/or antiinflammatory activity. For treating diseases in the region of the gastrointestinal tract, the EGFR inhibitors may also be administered in conjunction with substances having an effect on motility or secretion. These combinations may be administered either simultaneously or sequentially.

For example, in the case of a pancreatic tumor, the EGFR inhibitor may be advantageously combined with gemcitabine.

A promising combination is an EGFR inhibitor with a Ras inhibitor, e.g. an effective farnesyltransferase inhibitor (Cox and Der, 1997).

The EGFR inhibitors may be administered either on their own or in conjunction with other active substances by intravenous, subcutaneous, intramuscular, intrarectal, intraperitoneal or intranasal route, by inhalation or transdermally or orally, whilst aerosol formulations are particularly suitable for inhalation.

The EGFR inhibitors are generally used for warm-blooded vertebrates, particularly humans, in doses of 0.01-100 mg/kg of body weight, preferably 0.1-15 mg/kg. For administration they are formulated with one or more conventional inert carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethyleneglycol, propyleneglycol, stearylalcohol, carboxymethylcellulose or fatty substances such as hard fat or suitable mixtures thereof in conventional galenic preparations such as plain or coated tablets, capsules, powders, suspensions, solutions, sprays or suppositories.

Brief description of the figures:

Figure 1:    A:    Schematic representation of the K5-SOS-F transgene construct
             B:    Western blot analysis of transgenic skins
             C-G:  Phenotype of K5-SOS-F transgenic founders and offspring

Figure 2:    A, B:  Tumor incidence and volume of K5-SOS-F mice in the presence of different EGFR alleles

Figure 3:    EGFR-dependent transformation of immortalized fibroblasts by oncogenic SOS and Ras

Figure 4:    Increased apoptosis in K5-SOS-F papillomas and keratinocytes in the absence of a wild-type EGFR

Figure 5:    A:    SOS-, EGFR- expression and Akt and ERK activation in primary keratinocytes
             B:    Quantitative RT-PCR measuring HB-EGF and EGFR transcripts in primary keratinocytes and dermal fibroblasts
             C-E:  Histological sections of skin lesions

Figure 6:    Consequence of SOS-F expression in EGFR wild-type (wt) and mutant (wa2) epidermis

Figure 7:    Inhibition of a colon tumor expressing normal EGFR and a constituvively active Ras

Experimental Procedures

a) Generation of K5-SOS-F transgenic mice and establishment of transgenic lines in different EGFR backgrounds

A constitutively active, HA-tagged form of hSOS (SOS-F) (Aronheim et al., 1994) was excised from the plasmid vector as a HindIII fragment, blunt ended and inserted into a Sna BI site downstream of the K5 regulatory region (Murillas et al., 1995). The K5-SOS-F fragment was excised from the plasmid vector after digestion with the restriction enzymes Sall and Notl. Transgenic mice were generated by pronuclear injection of the purified K5-SOS-F DNA fragment into fertilized oocytes from (C57BL/6 x CBA) F1 mice. Founders were identified by Southern blot analysis (data not shown)

Founder 892A6* was first backcrossed to EGFR+/- mice of C57BL/6 background and the resulting EGFR+/-K5-SOS-F transgenics were further backcrossed to EGFRwa2/wa2 mice of LVC background (MRC-Chilton) to obtain wa2/+ and wa2/- mice harbouring the transgene. To monitor tumor incidence in the various EGFR backgrounds, offspring born from intercrosses between wa2/+ K5-SOS-F x wa2/wa2 and wa2/- K5-SOS-F x wa2/wa2 were kept and monitored over a year for the appearance of papillomas.

b) Histology, immunohistochemistry and TUNEL staining

Mouse tissues were fixed overnight in 4% paraformaldehyde, dehydrated and embedded in paraffin. 5 □ m sections were stained either with hematoxylin and eosin or processed further. Immunohistochemical staining for Ki67 (Novocastra, NCL-Ki67p, 1:1000) was performed using the ABC Staining Kit (Vector Laboratories) according to the manufacturer's recommendations. Immunostainings of mouse skins with Keratins were performed as described (Carroll et al., 1995).

TUNEL staining was performed using the in-situ cell death detection kit II (Boehringer Mannheim).

c) Isolation and culture of mouse keratinocytes and dermal fibroblasts

Mouse keratinocytes were isolated as previously described and cultured onto vitrogen-fibronectin coated dishes in low Calcium MEM (Sigma M8167) medium containing 8% chelated FCS (Carroll et al., 1995). Keratinocytes were induced to undergo terminal differentiation by suspension culture and recovered at the indicated timepoints as previously described (Gandarillas et al., 1999).

For the isolation of dermal fibroblasts mouse ears ears were split into dorsal and ventral side and placed in 1% Trypsin for 45 min at 37°C. The epidermis was separated from the dermis and the latter cut into small pieces and incubated at 37°C for 60 min in 1 mg/ml Collagenase/Dispase (Boehringer) with gentle stirring. Cells were filtered through a 70 μm Teflon mesh, centrifuged and resuspended in DMEM medium containing 10% FCS.

d) Immunoblotting analysis

Keratinocytes and minced tissues were homogenized in lysis buffer as previously described (Redemann et al., 1992). The lysates were cleared by centrifugation and processed for Western blot analysis as previously described (Sibilia and Wagner, 1995). The following antibodies were used: anti SOS1 (Transduction Laboratories), anti EGFR #1001 (Santa Cruz), anti ERK2 (Santa Cruz), anti phosphorylated ERK1/2 (Biolabs), anti Phosphotyrosine 4G10 (Upstate) anti phosphorylated Akt (Biolabs), anti Akt (Transduction Laboratories).

e) RNA isolation and RT-PCR

Total RNA was isolated from keratinocytes using the RNeasy Mini Kit (Quiagen). cDNA synthesis was performed with the Superscript Preamplification System (GibcoBRL) according to the manufacturer instructions. The following primers were used for RT-PCR analysis: EGFR1 GGAGGAAAAGAAAGTCTGCC, EGFR2 ATCGCACAGCACCAATCAGG; HB-EGF1 GCTGCCGTCGGTGATGCTGAAGC, HBEGF2 GATGACAAGAAGACAGACG; Tubulin1 CAACGTCAAGACGGCCGTGTG Tubulin2 GACAGAGGCAAACTGAGCACC. Transcripts were quantified with Light Cycler (Roche Diagnostics) using SYBR Green I and TaqStart Antibody (Clontech). Purified PCR amplicons were used to obtain absolute standard curves.

f) Nude mice skin grafting

Skin grafts were established on athymic mice as previously described (Dlugosz et al., 1997) using keratinocytes isolated from wa2/+ and wa2/-transgenic and non transgenic controls and dermal fibroblasts isolated from wa2/- mice. Mice were anesthetized and a silicone dome with a 2mm hole at its apex was positioned under the back skin. A slurry containing $4 \times 10^6$ primary dermal fibroblasts and $2 \times 10^6$ keratinocytes was applied to the silicone chamber. The grafting chambers were removed after 8 days and tumor growth was periodically monitored. 2 out of 3 grafts from wa2/+ K5-SOS-F keratinocytes attached and both started to form papillomas after 4 weeks. Only in 1 out of 3 wa2/- K5-SOS-F grafts a tiny papillomatous lesion could be detected after 7 weeks.

g) Fibroblast cell lines, retroviral infections and transfections

Primary mouse embryonic fibroblasts were isolated from wild-type and EGFR-/- E12.5 fetuses and immortalized according to the 3T3 protocol (Todaro et al., 1965). All fibroblasts were cultured in DMEM medium containing 10% FCS. The following retroviruses were employed for stable infection: pBabe-rasV12 (kindly provided by S. Lowe), pBabe-SOS-F (kindly provided by K. Matsuo) and pBabe (puromycin resistance) to infect EGFR+/+ and -/- 3T3 fibroblasts; pNTK-HERCD533 and pNTK (neomycin resistance) to infect NIH 3T3 cells (Redemann et al., 1992). Stable transfections were performed with the following plasmids: CMV-bcl-2, RSV-rasV12 and RSV-SOS-F cotransfected with RSV-hygro.

Apoptosis in NIH 3T3 fibroblasts was measured with the Flow-cytometer after labelling with AnnexinV-FITC/Propidium Iodide (PI) (Clontech) according to the manufacturer's recommendations.

h) Tumorigenicity assays

Fibroblasts were plated at $3 \times 10^5$ cells per 10 cm dish and infected or transfected with the above-described retroviruses and plasmid vectors 24 hr later. For the focus assay, cells were cultured in the same dishes without selection for 2 weeks. Cells were fixed and stained with 0.2% methylene blue (v/v in methanol). The number of macroscopically visible foci was estimated by visual examination.

For the tumorigenicity assay, the transfected or infected cells were selected with G418, puromycin or hygromycin for 1 week until all non-infected/transfected cells had died. $1 \times 10^6$ resistant cells were subcutaneously injected into 3-5 week old anaesthetized nude mice and the appearance of tumors was monitored.

Example 1

K5-SOS-F transgenic mice exhibit skin hyperplasia and develop papillomas

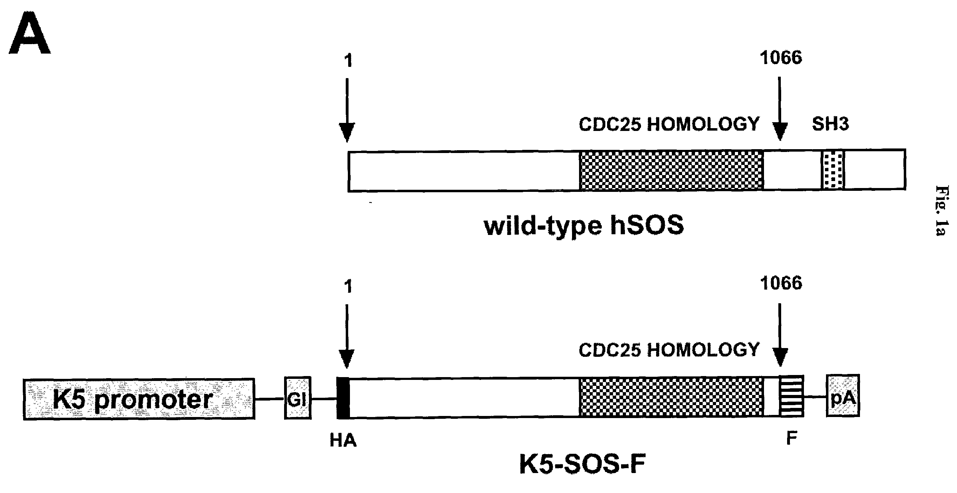

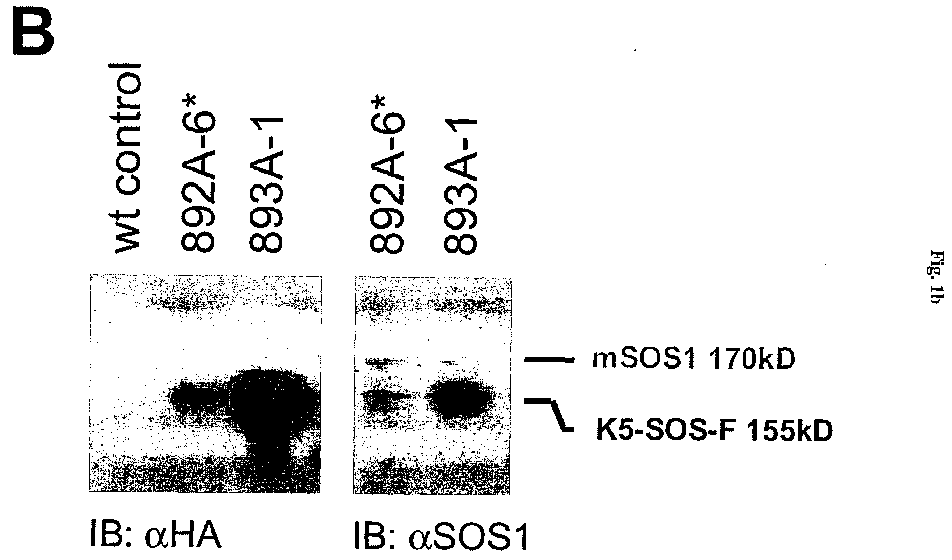

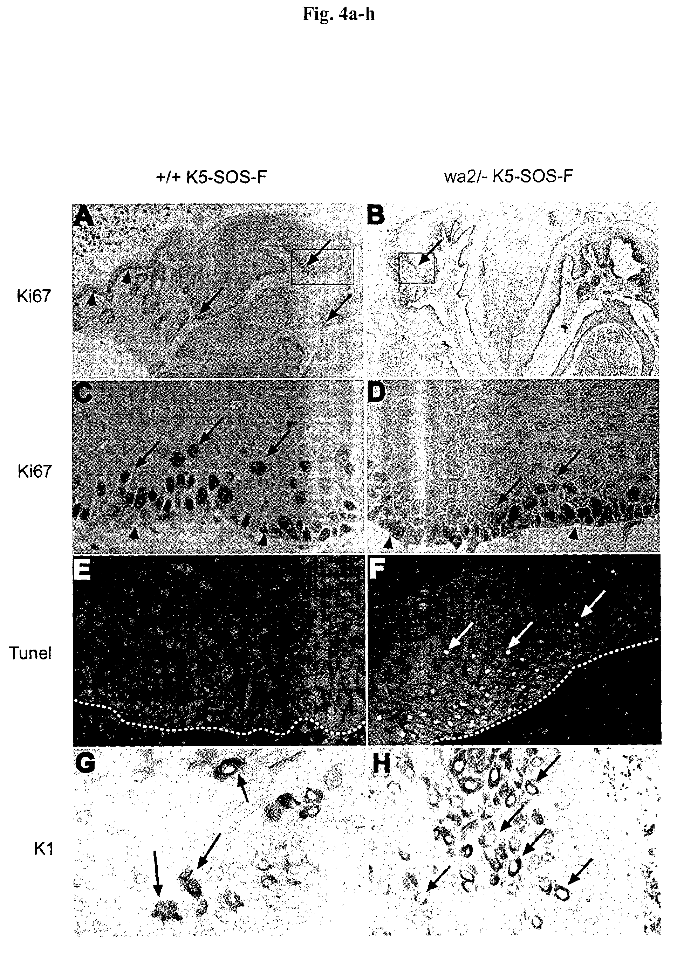

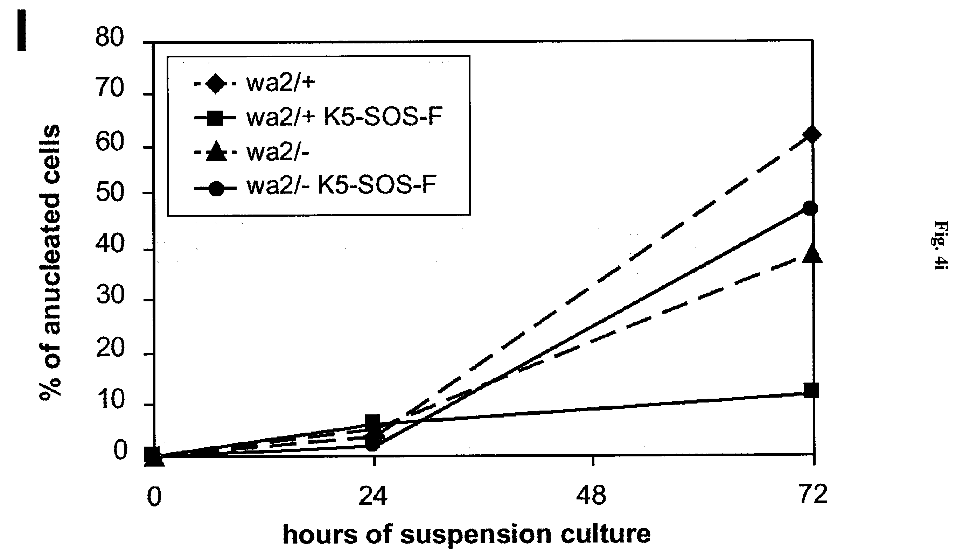

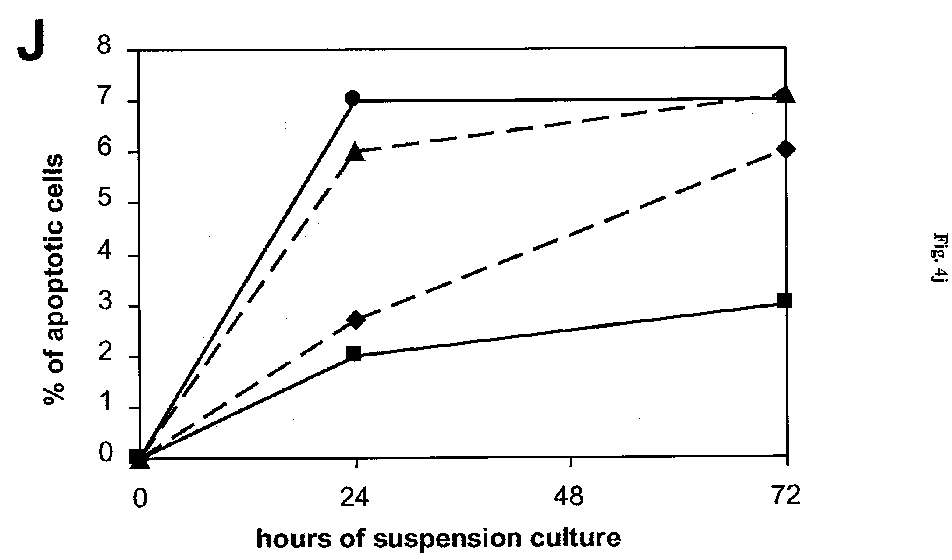

The full-length regulatory region of the K5 gene was used to express an activated form of human Son of Sevenless (SOS-F) in the basal cell compartment of stratified epithelia and in the outer root sheath of the hair follicles (Aronheim et al., 1994; Ramirez et al., 1994) (Fig. 1A). Four transgenic founder mice (892A-6*, 893A-1, 893B-1 and 893B-2) were obtained and SOS-F expression was found to be copy number-dependent since the founders harboring 3-5 (893A-1) or more than 50 copies of the transgene (893B-1 and 893B-2) showed the highest levels of expression of the SOS-F protein in the skin (Fig 1B and data not shown). The latter displayed dramatic skin hyperplasias on the entire body (Fig. 1D), open eyes (Fig. 1D), runted limbs (Fig. 1E) and died soon after birth probably due to the inability to breathe caused by a dramatic thickening of the tongue epithelium (data not shown). Founder 893A-1 carrying 3-5 copies of the transgene displayed no abnormalities at birth, but started to develop severe skin papillomas after 3 weeks and had to be sacrificed (data not shown). Founder 892A-6* carried only one or two copies of the transgene and showed low expression levels of the SOS-F protein similar to the endogenous SOS protein (Fig. 1B). Moreover, the SOS-F protein was expressed in the stomach, thymus, unaffected skin and papillomas, but was undetectable in other organs (data not shown). Founder 892A-6* which was used to establish the transgenic line started to develop papillomas after two weeks of age (Fig. 1F). All transgenic offspring developed severe skin alterations on the body and tail and histological examination revealed a hyperplastic epidermis displaying more than the usual 3 to 4 suprabasal cell layers present in controls (data not shown). Within the next 2-3 weeks, these hyperplastic lesions developed into macroscopically visible papillomatous structures with no or only very few hair follicles present in the affected areas (Fig. 1F,G, 4A). RNA in *situ* hybridization using a transgene-specific probe revealed expression of K5-SOS-F in all layers of hyperplastic skins and papillomas. Keratin 14 (K14), which is normally co-expressed with K5 in the basal layer, was also detectable throughout the epidermis of the hyperplastic lesions and papillomas (data not shown). These results show that expression of the transgene in the skin results in a hyperproliferative skin disease leading to hyperplasia and papilloma formation characterized by an expanded basal compartment.

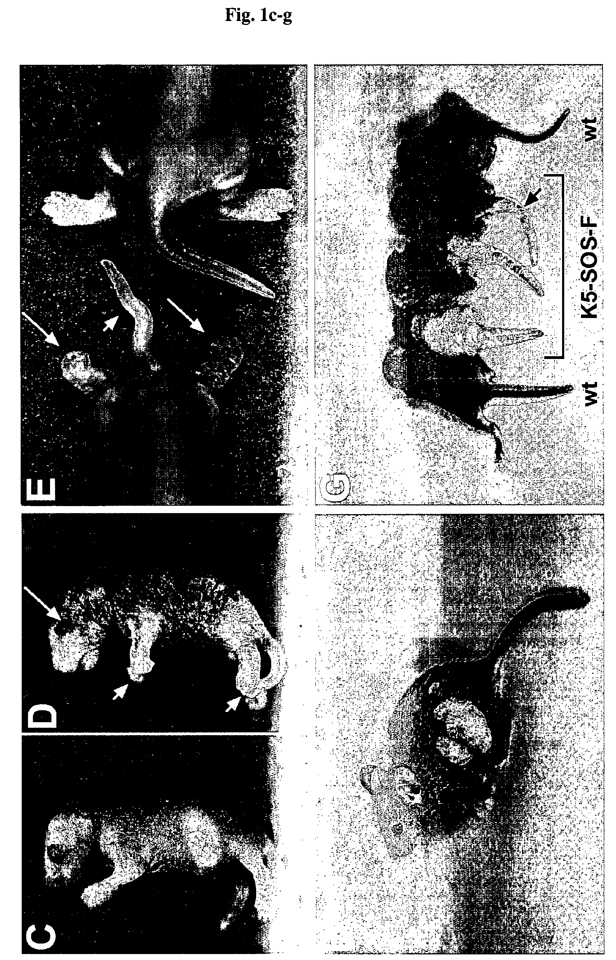

Figure 1 :(A) Schematic representation of the K5-SOS-F transgene construct. The upper line shows the structure of the wild-type human SOS (hSOS) cDNA with the position of the catalytic pocket denoted by the Cdc 25 homology domain and the proline-rich region binding the SH3 region of Grb2. The activated hSOS expressed from the Keratin 5 promoter harbors a HA-tag at the N-terminus, lacks the C-terminal region containing the Grb2 binding site and carries the c-Ha-ras farnesilation site (F) instead. B, Bam HI. (B) Western blot analysis of transgenic skins. Protein extracts from control skin and tumor biopsies of the two surviving founders 892A-6* and 893A-1 were immunoblotted with either anti-HA ($\alpha$HA) or anti-SOS ($\alpha$SOS1) antibodies as indicated (left panels). The positions of the 170kD endogenous mSOS1 and the 155 kD transgenic SOS-F are indicated.

(C-G) Phenotype of K5-SOS-F transgenic founders and offspring. (C) Normal and (D) transgenic littermate founder 893B-1 at birth. Note open eyes (big arrow), abnormal limbs (little arrows) and abnormal skin wrinkling and thickening in the transgenic pup, which died shortly after birth. (E) Runted limbs and kinky tail of the transgenic pup shown in (D) compared to the control littermate. Founder 893B-2 had very similar phenotypes and also died soon after birth (not shown). (F) Adult founder transgenic mouse 892A-6* showing multiple papillomas at different body sites. (G) Examples of 12 day old offspring from founder shown in (F) exhibiting extensive and localized areas of abnormal epidermis on the body and tail compared to two control littermates.

Example 2

Tumor development is impaired in a hypomorphic EGFRwa2/wa2 background

To explore a possible genetic interaction between SOS and EGFR, the K5-SOS-F transgene was bred into an EGFR null (-/-) or hypomorphic (wa2/wa2) background. Interestingly, skin tumor development was attenuated in the EGFR-/-. The transgene partially rescued the hair growth defects in the few mutants obtained and prolonged the lifespan up to 6 months (unpublished results). In contrast, in a wa2/wa2 or wa2/-background K5-SOS-F expression did not rescue the curly hair phenotype suggesting either that SOS controls hair growth but not hair follicle orientation or that the levels of expression of K5-SOS-F are not appropriate to rescue this phenotype.

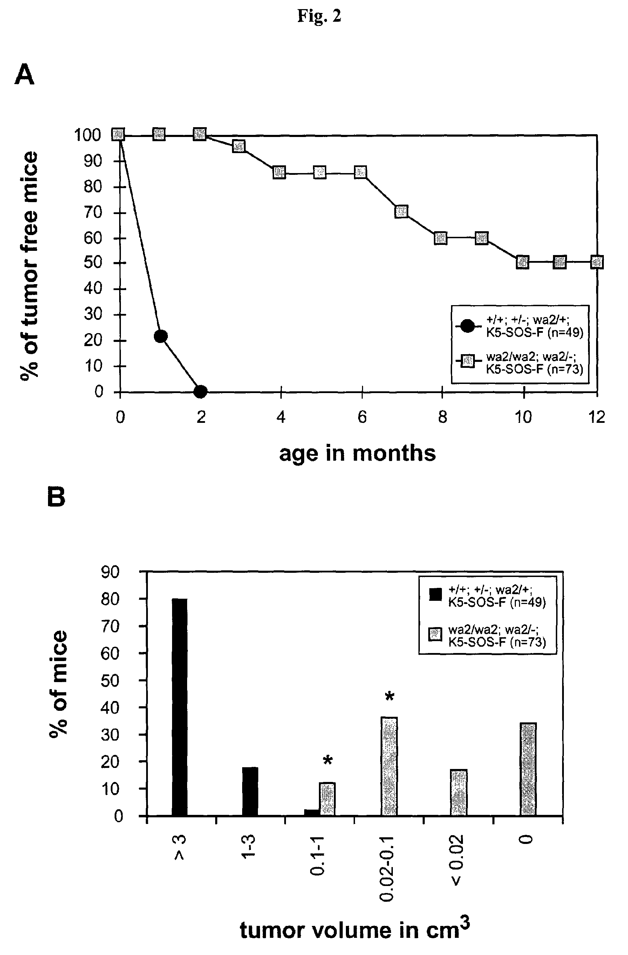

Skin tumor development was severely impaired in a wa2/wa2 or wa2/-transgenic background (Fig. 2A). After 6 months 85% of wa2/wa2 or wa2/-K5-SOS-F mice were still tumor-free, whereas 100% of +/+, +/- or wa2/+ K5-SOS-F mice had developed spontaneous tumors within the first two months (Fig. 2A). The average tumor volume was dramatically different between the groups. Whereas more than 90% of the papillomas in an EGFR wild-type background were bigger than 1 cm$^3$ after 3 weeks of age, tumor volume was drastically reduced in the presence of mutant EGFR alleles (Fig. 2B). In addition, the few lesions which developed in a mutant EGFR background only started to develop after an extended latency period (more than four months of age) and were frequently localized at the edges of ear-tag or tail-biopsy sites, suggesting that they were induced by wounding.

Figure 2 shows the tumor incidence (A) and volume (B) of K5-SOS-F mice in the presence of different EGFR alleles. 100% of EGFR+/+, +/-, and wa2/+ mice carrying the K5-SOS-F transgene develop large skin papillomas (B, black bars) within 2 months of age (A, black circles), whereas 50% of EGFR wa2/wa2 and wa2/- remain tumor free for more than 12 months (A, black squares) and never exceed the volume of 1 cm$^3$ (B, gray bars). Only skin lesions $\geq$ 0.02 cm$^3$ (marked by asterisks) were scored as tumors in (A). +/+, +/-, wa2/+ and wa2/wa2, wa2/- K5-SOS-F mice, respectively, were grouped since they showed similar tumor incidences and volumes.

Example 3

EGFR-dependent transformation of immortalized fibroblasts by oncogenic SOS and Ras

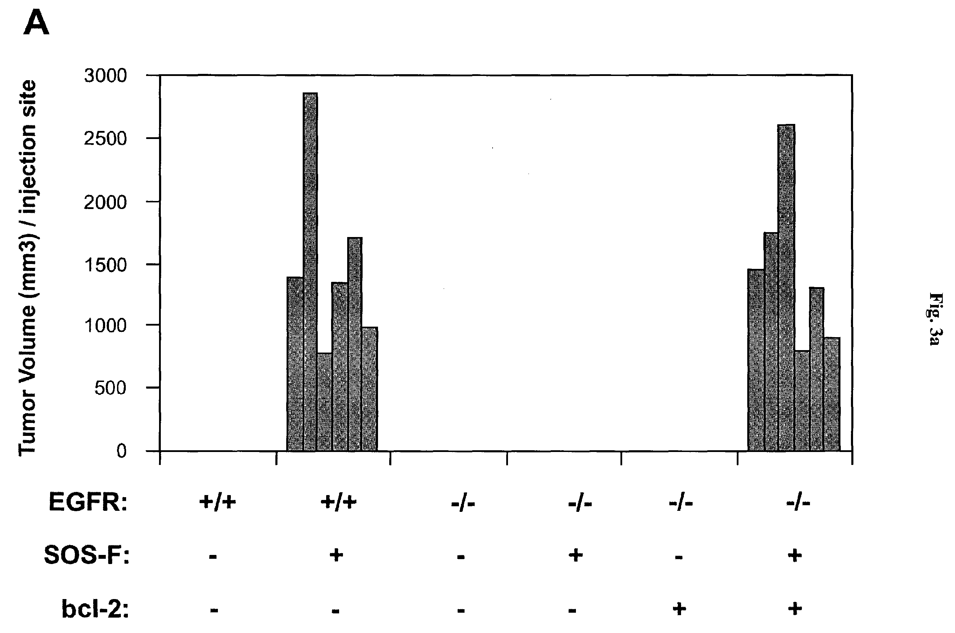

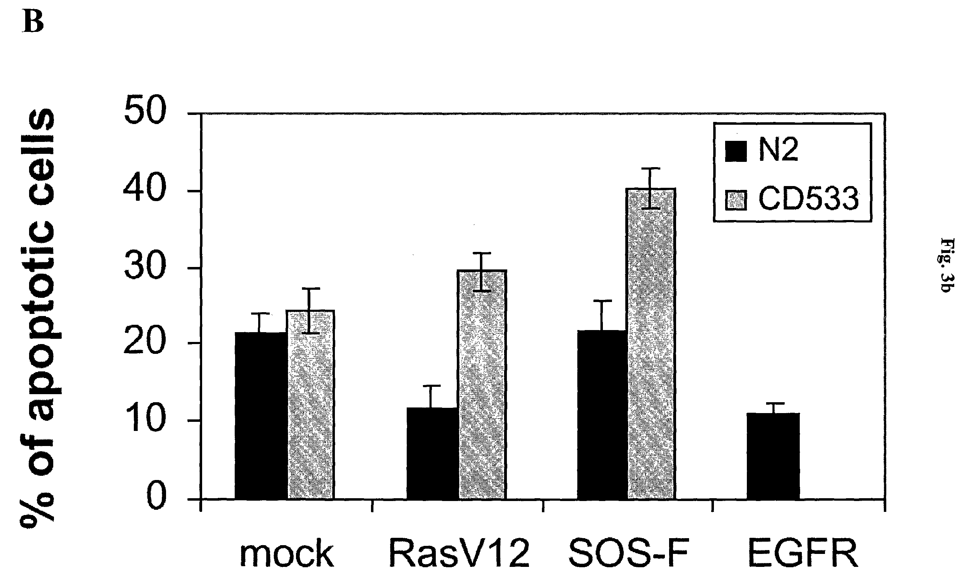

To gain insight into the molecular mechanism of EGFR-dependent transformation and to examine whether EGFR signaling was a prerequisite for oncogenic transformation by components of the Ras signaling pathway in other cell types, immortalized 3T3 fibroblasts isolated from EGFR-/-fetuses were employed. Their proliferation potential compared to wild-type was not affected (data not shown). Both, -/- and +/+ immortalized fibroblasts infected with a control virus were not tumorigenic, whereas wild-type cells expressing SOS-F efficiently formed tumors in nude mice (Fig. 3A). In contrast, fibroblasts lacking the EGFR failed to be transformed by SOS-F indicating that EGFR signaling is required for oncogenic transformation by SOS-F (Fig. 3A). Interestingly, expression of *bcl-2* restored tumor formation of -/- fibroblasts expressing SOS-F suggesting that the EGFR provides an anti-apoptotic signal (Fig. 3A).

To exclude the possibility that the resistance of -/- fibroblasts to SOS-F transformation was due to secondary genetic modifications, an independent fibroblast cell line expressing endogenous EGFR was analyzed. NIH3T3 fibroblasts were first infected with a retrovirus expressing a dominant negative EGFR mutant (CD533), and then transfected with plasmids encoding SOS-F or *ras*V12 (Table). Both SOS-F and rasV12 efficiently transformed NIH3T3 cells as judged by focus formation *in vitro* and tumor formation in nude mice (Table). Inhibition of EGFR function by expression of CD533 completely abolished transformation by SOS-F (Table). Moreover, CD533-expression almost completely abolished transformation by rasV12 suggesting that EGFR is not only required for oncogenic transformation by SOS-F, but also for transformation by other components of the Ras signaling pathway (Table).

When apoptosis was measured in these transfected fibroblasts, no difference in the percentage of apoptotic cells was detectable between controls and cells expressing a dominant negative EGFR (Fig. 3B). Strikingly, in SOS-F- or rasV12-transfected NIH3T3 fibroblasts, inhibition of EGFR function by CD533 resulted in a marked increase in the number of apoptotic cells (Fig. 3B). These results suggest that EGFR plays an important role in anti-apoptotic signaling in oncogenic transformation by components of the Ras signaling pathway.

Figure 3 :(A) Tumor formation in nude mice by EGFR+/+ and -/- mouse embryonic fibroblast cell lines infected or transfected with various constructs. Fibroblasts were infected with a retrovirus encoding SOS-F or an empty virus. Successively, -/- SOS-F fibroblasts were transfected with bcl-2. Tumorigenicity was assayed after subcutaneous injection of 1 x 10$^6$ cells into nude mice. There was no difference in the infection and transfection frequency between wild-type and -/- cells. (Table) Transforming activity of NIH 3T3 cells first stably infected with an empty (N2) retrovirus or with a retrovirus harboring a dominant negative EGFR (CD533) and successively with an empty plasmid (mock) or constructs encoding SOS-F or rasV12. Transformation was measured by Focus Assay or by the ability to form tumors in nude mice. (B) Increased apoptosis in SOS-F or rasV12 transformed NIH 3T3 cells in the presence of a dominant negative EGFR (CD533) as measured by Flow-cytometry after AnnexinV-FITC/Propidium Iodide (PI) staining. The bar diagram shows the % of apoptotic cells as the sum of AnnexinV single and AnnexinV/PI double positive cells. Results of one experiment are shown and the data represent the mean $\pm$SD of the analysis of three independent cultures of the respective genotypes. A second independent experiment gave very similar results (data not shown). Similar differences in apoptosis among the various cell lines were observed when fibroblasts were grown in 10% serum (data not shown). NIH 3T3 fibroblasts infected with a retrovirus expressing the wild-type human EGFR (EGFR) were taken as control. The expression of the respective transfected or infected proteins was verified by Western blot analysis (data not shown).

Example 4

Increased apoptosis in wa2/- K5-SOS-F transgenic papillomas and primary keratinocytes

Although wa2/- or wa2/wa2 transgenic papillomas were much smaller in size and developed much later (Fig. 2A, B), histologically they appeared very similar to the ones observed in a +/+ or wa2/+ background (Fig. 4A, B). Staining with the proliferation marker Ki67 showed that the majority of basal cells were proliferating and that the number of proliferating cells was increased to a similar extent in +/+ and wa2/- transgenic papillomas (Fig. 4A-D). In both backgrounds the proliferating cells, which are normally confined to the basal layer, had expanded to the suprabasal compartments (arrows in Fig. 4C, D). In contrast, in adjacent non-affected transgenic skin, Ki67-positive cells were present in the basal layer (Fig. 4A, arrowheads). When skin tumor sections were labeled with the TUNEL technique, a significant increase in the number of apoptotic cells was detected in the basal and suprabasal layers of wa2/- K5-SOS-F papillomas (Fig. 4F, arrows). Quantification of apoptosis on multiple sections of different tumors revealed a 3-fold increase in the number of apoptotic cells in wa2/-papillomas (145.3 ± 13.6) compared to wild-type tumors (50.6 ± 9.6). To assess the effect of K5-SOS-F expression on terminal differentiation, tumors of both backgrounds were stained for Keratin 1 which is normally limited to the suprabasal layer of the epidermis. Whereas the expression of K1 was almost absent in +/+ papillomas, a significantly increased number of differentiated cells was still present in wa2/- tumors (Fig. 4G, H) suggesting that signaling by the wild-type EGFR might negatively affect terminal differentiation.

The survival capacity of primary transgenic keratinocytes was followed during differentiation *in vitro,* which was induced by deprivation of cell anchorage by means of suspension culture. Consistent with a differentiation defect, the number of anucleated cells was considerably lower in wa2/+ K5-SOS-F keratinocytes after 72h of suspension culture compared to cells of all three other genotypes (Fig. 4I). After 72h of suspension culture a similar number of apoptotic cells was observed in non-transgenic wa2/+ and wa2/- control keratinocytes (Fig. 4J). In contrast, K5-SOS-F keratinocytes in the presence of a wild-type EGFR displayed only half the number of apoptotic cells compared to transgenic keratinocytes of a hypomorphic EGFR background (Fig. 4J). These results indicate that expression of K5-SOS-F renders basal keratinocytes prone to hyperproliferation, but that a functional EGFR is required to negatively regulate keratinocyte differentiation and to provide a survival signal for tumor development.

Figure 4: Increased apoptosis in K5-SOS-F papillomas and keratinocytes in the absence of a wild-type EGFR. (A-D) Anti-Ki67 immunostaining of papillomas isolated from a 3-week-old +/+ (A, C) and a 10 months-old wa2/-(B, D) transgenic mouse. The number of proliferating cells which have extended to the suprabasal layers is increased to a similar extent in +/+ (arrows in A, C) and wa2/- (arrows in B, D) transgenic papillomas. Note that the majority of basal cells are also proliferating in the non-affected transgenic skin (arrowhead in A). Hair follicles are absent in the affected areas. (C, D) Higher magnifications of the boxed areas shown in A and B. Arrowheads point to basal cells. (E, F) TUNEL staining (green) of adjacent sections to A and B showing a significantly higher number of apoptotic cells in the basal and suprabasal compartments of transgenic papillomas in the absence of a wild-type EGFR (arrows in F). Propidium Iodide was used as a nuclear counterstain. The dotted lines in E and F delineate the basal membrane. (G, H) Immunostaining for Keratin 1. Arrows point to differentiated cells which are significantly increased in number in wa2/- tumors (H). Impaired differentiation (I) and reduced apoptosis (J) in primary keratinocytes of wa2/+ K5-SOS-F mice. In vitro differentiation of primary keratinocytes was induced by suspension culture for the indicated times. Cells suspended in methyl-cellulose were harvested and air-dried on coverslips and scored for the following parameters: (I) Differentiation by the number of anucleated cells, (J) Apoptosis by the number of small cells with condensed, fragmented nuclei as judged by DAPI/TUNEL double staining or hematoxylin and eosin staining. Symbols in I and J correspond to the same genotypes. Data in I and J represent the respective percentages after counting 300 cells from randomly chosen fields. A second independent experiment gave similar results (data not shown). (Original magnifications: A, B 10x; C-H 40x)

Example 5

Impaired Akt but normal ERK activation in wa2/- K5-SOS-F keratinocytes

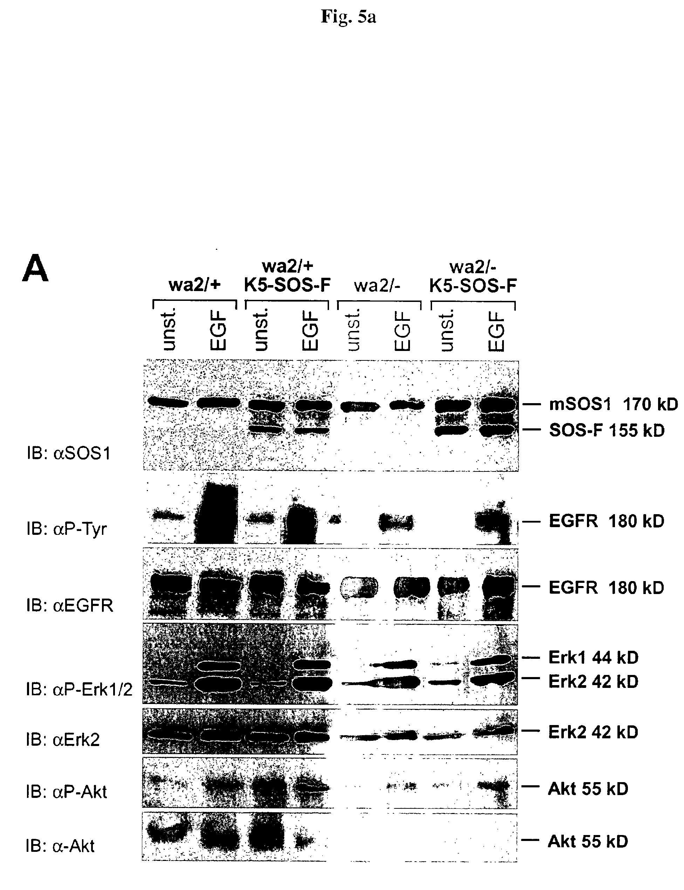

To exclude the possibility that lack of tumor formation in a hypomorphic EGFR background was due to reduced transgene expression or impaired EGFR activation, primary basal keratinocyte cultures established from wa2/+ and wa2/- transgenic mice and non-transgenic littermates were analyzed for protein expression. Western blot analysis using antibodies against SOS1 confirmed that the levels of SOS-F protein were similar to the levels of the endogenous SOS protein and comparable in the wa2/+ and wa2/- background (Fig. 5A). Likewise, the levels of EGFR protein were not different between the various genotypes and EGF stimulation led to phosphorylation of the EGFR, although this was slightly reduced in a wa2/wa2 background (Fig. 5A, Luetteke et al., 1994). Activation of Aktkinase, which positively regulates a pathway leading to cell survival in epithelial cells, was significantly reduced in wa2/- K5-SOS-F keratinocytes (Fig. 5A). In contrast, ERK1/2 phosphorylation after EGF treatment was comparable and not influenced by the presence of the hypomorphic wa2 EGFR allele (Fig. 5A). These results demonstrate that whereas ERK activation is not affected, Akt phosphorylation is impaired in wa2/-transgenic keratinocytes, thus providing a molecular explanation for the survival function of the EGFR.

To investigate if the presence of K5-SOS-F was leading to increased expression of EGFR ligands, thereby activating an autocrine loop, total RNA was extracted from different keratinocyte cultures. Quantitative RT-PCR analysis revealed that EGF stimulation resulted in an elevation of HB-EGF mRNA transcription and that HB-EGF induction was significantly enhanced by the expression of K5-SOS-F (Fig 5B). The transcription of the EGFR itself was not significantly altered (Fig 5B). These data suggest that an autocrine loop might be activated, but since the induction of HB-EGF expression is increased in both wa2/+ and wa2/- K5-SOS-F keratinocytes, this alone can not account for the differences in tumor formation observed *in vivo.*

We next investigated whether the lack of skin tumor formation in a wa2/-background could be attributed to differences between wa2/+ versus wa2/-dermis. For this purpose, primary dermal fibroblasts were isolated from mice of all four genotypes. Quantitative RT-PCR analysis did not reveal significant differences in expression levels of HB-EGF and EGFR in all fibroblast groups, which were not affected by the presence of K5-SOS-F since the latter is not expressed in the dermis (Fig. 5B).

Figure 5: (A) SOS-, EGFR- expression and Akt and ERK activation in primary keratinocytes. Keratinocytes of various genotypes were starved for 48h in 0.5% serum and stimulated for 5 minutes with 20ng/ml EGF. Protein extracts were separated on a 8% SDS-polyacrylamide gel, transferred to a membrane and immunoblotted with the indicated antibodies. (B) Quantitative RT-PCR measuring HB-EGF and EGFR transcripts in primary keratinocytes and dermal fibroblasts. Cells of the respective genotypes were starved for 48h in 0.5% serum and stimulated for 4 hours with 20ng/ml EGF. (C-E) Histological sections of skin lesions developing from wa2/+ (C) wa2/+ K5-SOS-F (D) and wa2/- K5-SOS-F (E) keratinocytes grafted in combination with wa2/- dermal fibroblasts onto the back of nude mice. Note the big papilloma-like structure of the tumor derived from wa2/+ K5-SOS-F keratinocytes (D) versus the small lesion observed in wa2/- K5-SOS-F keratinocytes (E). Arrows in E point to hyperplastic papilloma-like structures. No hyperplasias were detected in the control (C). Arrow in C points to the site of wound closure in the grafted skin. All the lesions were isolated 7 weeks after grafting. (Original magnifications: C-E 5x)

Example 6

A cell-autonomous requirement for the EGFR in keratinocytes

To analyze whether the EGFR was required cell-autonomously in transgenic keratinocytes and to demonstrate that mutant dermis was able to support tumor growth, grafting experiments were performed with transgenic keratinocytes in combination with wa2/- dermal fibroblasts. In grafts from wa2/+ K5-SOS-F keratinocytes tumors became apparent after 4 weeks while no tumors could be detected in grafts established with wa2/-K5-SOS-F keratinocytes. Within the next 3 weeks the tumors derived from wa2/+ K5-SOS-F keratinocytes increased in size and appeared macroscopically and histologically as typical papillomas (Fig 5D). After 7 weeks, one of the grafts from wa2/- K5-SOS-F keratinocytes appeared as a small papillomatous lesion which was >20 times smaller than the papillomas derived from wa2/+ transgenic keratinocytes. Histological examination of the lesion derived from wa2/- K5-SOS-F keratinocytes revealed a hyperplastic epidermis (Fig. 5E arrows) whereas no signs of hyperplasia could be detected in control grafts established with wa2/+ keratinocytes (Fig. 5C). These results indicate that a functional EGFR is needed in keratinocytes to promote tumor development in K5-SOS-F mice and that papilloma formation is most likely not influenced by the presence or absence of a functional EGFR in the dermis.

Figure 6: Consequence of SOS-F expression in EGFR wild-type (wt) and mutant (wa2) epidermis. Keratinocyte survival, proliferation and differentiation are tightly regulated processes during skin development. Whereas Ras induction is essential for keratinocyte proliferation, EGFR signaling in basal keratinocytes activates an anti-apoptotic pathway possibly via Akt and inhibits keratinocyte differentiation in a SOS/Ras-dependent and/or -independent manner. (A) Expression of a dominant SOS-F activates Ras and therefore leads to increased proliferation and reduced differentiation of keratinocytes. In addition, the wild-type EGFR acts as a potent survival signal leading to skin tumor development. (B) In the presence of a hypomorphic EGFR the survival pathway downstream of the EGFR is not sufficiently activated and as a consequence tumors can not develop.

Example 7

Inhibition of a colon tumor expressing normal EGFR and a constituvively active Ras

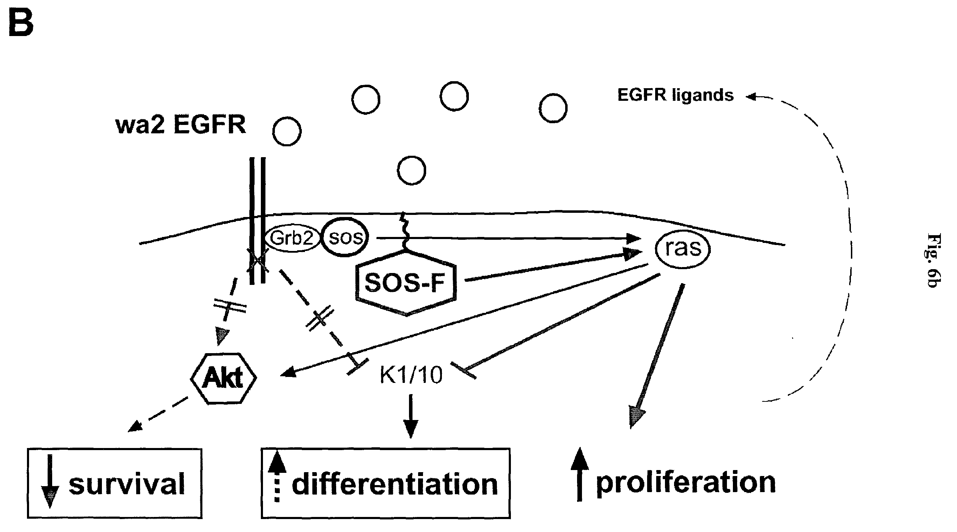

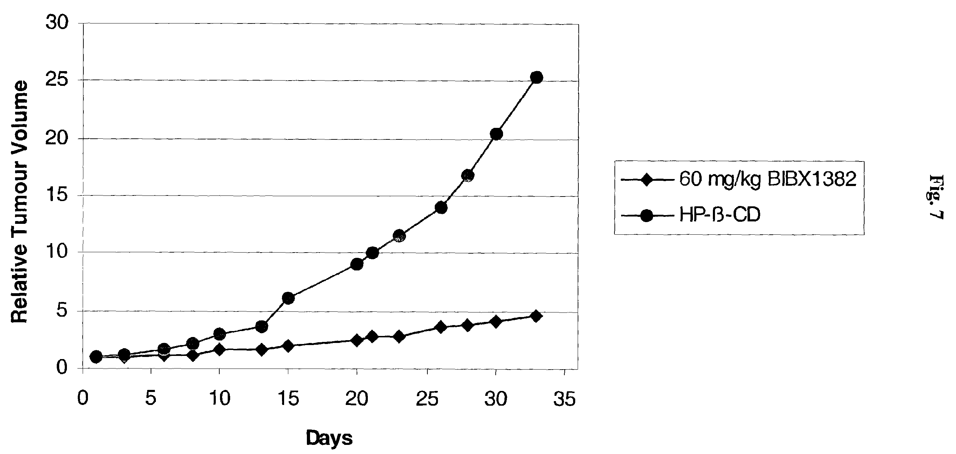

Pieces of the colon adenocarcinoma CG736 were directly transfered from a patient to nude mice and passaged as subcutaneous xenografts according to standard procedures. It was observed that growth of subcutaneous tumour xenografts was sensitive to the EGFR inhibitor BIBX1382 (4-((3-chloro-4-fluoro-phenyl)amino)-6-(1-methyl-4-piperidinyl-amino)-pyrimido(5,4d)pyrimidine). Once daily oral treatment at 60 mg/kg/d over 5 weeks resulted in a significant reduction of the tumour growth rate (median increase of tumour volume in the test group (BIBX1382 in 25% Hydroxipropyl-β-cyclodextrin HP-β-CD) was 5-fold as opposed to 20-fold in the control group receiving HP-β-CD only). Tumour sensitivity was maintained through >10 passages. Tumour cells expressed normal levels of apparently wtEGFR as judged by amounts and sizes of transcripts. Testing cells derived from colon adenocarcinoma CG736 in a microphysiometer showed that cells could be stimulated by EGF at concentrations of <10 ng/ml as evident from EGF-induced medium acidification. EGFR-signaling in these cells was inhibited by BIBX1382 with an IC50 of 2 µM. Importantly, a mutation of Ras at codon 13 (GGC→GAC, Gly→Asp) was detected in tumour samples from early and late passages. Taken together, these observations show that a tumour coexpressing apparently normal EGFR and a constitutively active Ras displays sensitivity to an EGFR inhibitor. The results are shown in Fig. 7.

Example 8

Galenic formulations of the EGFR inhibitor BIBX1382

a) Coated tablets containing 75 mg of BIBX1382

1 tablet core contains:

| | |
|---|---|
| BIBX1382 | 75.0 mg |
| calcium phosphate | 93.0 mg |
| corn starch | 35.5 mg |
| polyvinylpyrrolidone | 10.0 mg |
| hydroxypropylmethylcellulose | 15.0 mg |
| magnesium stearate | 1.5 mg |
| | 230.0 mg |

Preparation:

BIBX1382 is mixed with calcium phosphate, corn starch, polyvinylpyrrolidone, hydroxypropylmethylcellulose and half the specified amount of magnesium stearate. Blanks 13 mm in diameter are produced in a tablet-making machine and these are then rubbed through a screen with a mesh size of 1.5 mm using a suitable machine and mixed with the rest of the magnesium stearate. This granulate is compressed in a tablet-making machine to form tablets of the desired shape.

| | |
|---|---|
| Weight of core | 230 mg |
| die | 9 mm, convex |

The tablet cores thus produced are coated with a film consisting essentially of hydroxypropylmethylcellulose. The finished film-coated tablets are polished with beeswax.

| | |
|---|---|
| Weight of coated tablet | 245 mg. |

b) Tablets containing 100 mg of BIBX1382

Composition:

1 tablet contains:

| | |
|---|---|
| BIBX1382 | 100.0 mg |
| lactose | 80.0 mg |
| corn starch | 34.0 mg |
| polyvinylpyrrolidone | 4.0 mg |
| magnesium stearate | 2.0 mg |
| | 220.0 mg |

Method of Preparation:

BIBX1382, lactose and starch are mixed together and uniformly moistened with an aqueous solution of the polyvinylpyrrolidone. After the moist composition has been screened (2.0 mm mesh size) and dried in a rack-type drier at 50°C it is screened again (1.5 mm mesh size) and the lubricant is added. The finished mixture is compressed to form tablets.

| Weight of tablet | 220 mg |
|---|---|
| Diameter | 10 mm, biplanar, facetted on both sides and notched on one side. |

c) Tablets containing 150 mg of BIBX1382

Composition:

1 tablet contains:

| BIBX1382 | 150.0 mg |
|---|---|
| powdered lactose | 89.0 mg |
| corn starch | 40.0 mg |
| colloidal silica | 10.0 mg |
| polyvinylpyrrolidone | 10.0 mg |
| magnesium stearate | 1.0 mg |
| | 300.0 mg |

Preparation:

BIBX1382 mixed with lactose, corn starch and silica is moistened with a 20% aqueous polyvinylpyrrolidone solution and passed through a screen with a mesh size of 1.5 mm. The granules, dried at 45°C, are passed through the same screen again and mixed with the specified amount of magnesium stearate. Tablets are pressed from the mixture.

| Weight of tablet | 300 mg |
|---|---|
| die | 10 mm, flat |

d) Hard gelatine capsules containing 150 mg of BIBX1382

1 capsule contains:

| BIBX1382 | 150.0 mg |
|---|---|
| corn starch (dried) approx. | 180.0 mg |
| lactose (powdered) approx. | 87.0 mg |
| magnesium stearate approx. | 3.0 mg |
| | 420.0 mg |

Preparation:

BIBX1382 is mixed with the excipients, passed through a screen with a mesh size of 0.75 mm and homogeneously mixed using a suitable apparatus. The finished mixture is packed into size 1 hard gelatine capsules.

| Capsule filling | approx. 320 mg |
|---|---|
| Capsule shell | size 1 hard gelatine capsule. |

Table

| NIH 3T3 | | Transformation Focus Assay | Tumors in nude mice |
|---|---|---|---|
| **1st infection** | **2nd transfection** | | |
| N2 (control) | mock | - | no |

Table (continued)

| NIH 3T3 | | Transformation Focus Assay | Tumors in nude mice |
|---|---|---|---|
| 1st infection | 2nd transfection | | |
| N2 (control) | SOS-F | ++ | yes |
| N2 (control) | RasV12 | ++++ | yes |
| | | | |
| CD533 (DN EGFR) | mock | - | no |
| CD533 (DN EGFR) | SOS-F | - | no |
| CD533 (DN EGFR) | RasV12 | +/- | no |

References

Aronheim, A., Engelberg, D., Li, N., al-Alawi, N., Schlessinger, J., and Karin, M. (1994). Membrane targeting of the nucleotide exchange factor Sos is sufficient for activating the Ras signaling pathway. Cell 78, 949-61.

Bailleul, B., Surani, M. A., White, S., Barton, S. C., Brown, K., Blessing, M., Jorcano, J., and Balmain, A. (1990). Skin hyperkeratosis and papilloma formation in transgenic mice expressing a ras oncogene from a suprabasal keratin promoter. Cell 62, 697-708.

Barbacid, M. (1990). ras oncogenes: their role in neoplasia. Eur. J. Clin. Invest. 20, 225-235.

Bergmann, A., Agapite, J., McCall, K., and Steller, H. (1998). The Drosophila gene hid is a direct molecular target of Ras-dependent survival signaling. Cell 95, 331-41.

Brown, K., Quintanilla, M., Ramsden, M., Kerr, I. B., Young, S., and Balmain, A. (1986). v-ras genes from Harvey and BALB murine sarcoma viruses can act as initiators of two-stage mouse skin carcinogenesis. Cell 46, 447-456.

Bos, (1988), Mutation Res. 195: 255-271

Bown, K., Strathdee, D., Bryson, S., Lambie, W., and Balmain, A. (1998). The malignant capacity of skin tumours induced by expression of a mutant H-ras transgene depends on the cell type targeted. Curr Biol 8, 516-24.

Carroll, J. M., Romero, M. R., and Watt, F. M. (1995). Suprabasal integrin expression in the epidermis of transgenic mice results in developmental defects and a phenotype resembling psoriasis. Cell 83, 957-968.

Cox, A. D. and Der, C.J. (1997). Biochim. Biophys. Acta 1333, F51-f71

Denning, M. F., Dlugosz, A. A., Howett, M. K., and Yuspa, S. H. (1993). Expression of an oncogenic rasHa gene in murine keratinocytes induces tyrosine phosphorylation and reduced activity of protein kinase C delta. J Biol Chem 268, 26079-81.

Denning, M. F., Dlugosz, A. A., Threadgill, D. W., Magnuson, T., and Yuspa, S. H. (1996). Activation of the epidermal growth factor receptor signal transduction pathway stimulates tyrosine phosphorylation of protein kinase C delta. J Biol Chem 271, 5325-31.

Derynck, R. (1992). The physiology of transforming growth factor-alpha. Adv Cancer Res 58, 27-52.

Dlugosz, A. A., Cheng, C., Williams, E. K., Dharia, A. G., Denning, M. F., and Yuspa, S. H. (1994). Alterations in murine keratinocyte differentiation induced by activated rasHa genes are mediated by protein kinase C-alpha. Cancer Res 54, 6413-20.

Dlugosz, A. A., Hansen, L., Cheng, C., Alexander, N., Denning, M. F., Threadgill, D. W., Magnuson, T., Coffey, R. J., Jr., and Yuspa, S. H. (1997). Targeted disruption of the epidermal growth factor receptor impairs growth of squamous papillomas expressing the v-ras(Ha) oncogene but does not block in vitro keratinocyte responses to oncogenic ras. Cancer Res 57, 3180-8.

Dominey, A. M., Wang, X.-J., King, L. E., Nanney, L. B., Gagne, T. A., Sellheyer, K., Bundman, D. S., Longley, M. A., Rothnagel, J. A., Greenhalgh, D. A., and Roop, D. R. (1993). Targeted overexpression of transforming growth factor alpha in the epidermis of transgenic mice elicits hyperplasia, hyperkeratosis, and spontaneous, squamous papillomas. Cell Growth & Diff 4, 1071-1082.

Downward, J. (1998). Ras signalling and apoptosis. Curr Opin Genet Dev 8, 49-54.

Earp, H. S., Dawson, T. L., Li, X., and Yu, H. (1995). Heterodimerization and functional interaction between EGF receptor family members: a new signaling paradigm with implications for breast cancer research. Breast Cancer Res Treat 35, 115-32.

Fong C.J., Sherwood ER, Mendelsohn J, Lee C, Kozlowski JM, Cancer Res. 1992 Nov 1;52(21):5887-92

Fowler, K. J., Walker, F., Alexander, W., Hibbs, M. L., Nice, E. C., Bohmer, R. M., Mann, G. B., Thumwood, C., Maglitto, R., Danks, J. A., and et al. (1995). A mutation in the epidermal growth factor receptor in waved-2 mice has a profound effect on receptor biochemistry that results in impaired lactation. Proc Natl Acad Sci U S A 92, 1465-9.

Fuchs, E. (1990). Epidermal differentiation: the bare essentials. J Cell Biol 111, 2807-14.

# EP 1 170 011 A1

Fuchs, E. (1992). Genetic skin disorders of keratin. J Invest Dermatol 99, 671-4.

Gandarillas, A., Goldsmith, L. A., Gschmeissner, S., Leigh, I. M., and Watt, F. M. (1999). Evidence that apoptosis and terminal differentiation of epidermal keratinocytes are distinct processes. Exp Dermatol 8, 71-9.

Gangarosa, L. M., Sizemore, N., Graves-Deal, R., Oldham, S. M., Der, C. J., and Coffey, R. J. (1997). A raf-independent epidermal growth factor receptor autocrine loop is necessary for Ras transformation of rat intestinal epithelial cells. J Biol Chem 272, 18926-31.

Hansen, L. A., Alexander, N., Hogan, M. E., Sundberg, J. P., Dlugosz, A., Threadgill, D. W., Magnuson, T., and Yuspa, S. H. (1997). Genetically null mice reveal a central role for epidermal growth factor receptor in the differentiation of the hair follicle and normal hair development. Am J Pathol 150, 1959-75.

Hasegawa, Y., et al., (1995), Oncogene, 10, 1441-1445

Jones, P. H., and Watt, F. M. (1993). Separation of human epidermal stem cells from transit amplifying cells on the basis of differences in integrin function and expression. Cell 73, 713-24.

Kauffmann-Zeh, A., Rodriguez-Viciana, P., Ulrich, E., Gilbert, C., Coffer, P., Downward, J., and Evan, G. (1997). Suppression of c-Myc-induced apoptosis by Ras signalling through PI(3)K and PKB. Nature 385, 544-8.

Kiaris, H., et al., (1995), British Journal of Cancer, 72, 123-128

King, L. E., Jr., Gates, R. E., Stoscheck, C. M., and Nanney, L. B. (1990). The EGF/TGF alpha receptor in skin. J Invest Dermatol 94, 164S-170S.

Kurada, P., and White, K. (1998). Ras promotes cell survival in Drosophila by downregulating hid expression. Cell 95, 319-29.

Lehman, T.A., et al., (1996), Analytical Biochemistry, 239, 153-159

Lemmon, M. A., and Schlessinger, J. (1994). Regulation of signal transduction and signal diversity by receptor oligomerization. Trends Biochem Sci 19, 459-63.

Luetteke, N. C., Phillips, H. K., Qiu, T. H., Copeland, N. G., Earp, H. S., Jenkins, N. A., and Lee, D. C. (1994). The mouse waved-2 phenotype results from a point mutation in the EGF receptor tyrosine kinase. Genes Dev 8, 399-413.

Luetteke, N. C., Qiu, T. H., Peiffer, R. L., Oliver, P., Smithies, O., and Lee, D. C. (1993). TGF alpha deficiency results in hair follicle and eye abnormalities in targeted and waved-1 mice. Cell 73, 263-78.

Mann, G. B., Fowler, K. J., Gabriel, A., Nice, E. C., Williams, R. L., and Dunn, A. R. (1993). Mice with a null mutation of the TGF alpha gene have abnormal skin architecture, wavy hair, and curly whiskers and often develop corneal inflammation. Cell 73, 249-61.

McCarthy, S. A., Samuels, M. L., Pritchard, C. A., Abraham, J. A., and McMahon, M. (1995). Rapid induction of heparin-binding epidermal growth factor/diphtheria toxin receptor expression by Raf and Ras oncogenes. Genes Dev 9, 1953-64.

McMahon et al., Current Opinion in Drug Discovery & Development 1998 1 (2): 131-146

Miettinen, P. J., Berger, J. E., Meneses, J., Phung, Y., Pedersen, R. A., Werb, Z., and Derynck, R. (1995). Epithelial immaturity and multiorgan failure in mice lacking epidermal growth factor receptor. Nature 376, 337-41.

Modjtahedi H, Dean C. The receptor for EGF and its ligands: expression, prognostic value and target for therapy in cancer. Int. J. Oncology 4: 277-296 (1994)

Modjtahedi H, Komurasaki T, Toyoda H, Dean C. (1998). Int J Cancer. Jan 19;75(2):310-6.

Murillas, R., Larcher, F., Conti, C. J., Santos, M., Ullrich, A., and Jorcano, J. L. (1995). Expression of a dominant negative mutant of epidermal growth factor receptor in the epidermis of transgenic mice elicits striking alterations in hair follicle development and skin structure. Embo J 14, 5216-23.

Nagane, M., Levitzki, A., Gazit, A., Cavenee, W. K., and Huang, H. J. (1998). Drug resistance of human glioblastoma cells conferred by a tumor-specific mutant epidermal growth factor receptor through modulation of Bcl-XL and caspase-3-like proteases. Proc Natl Acad Sci U S A 95, 5724-9.

Normanno N, Tortora G, De Luca A, Pomatico G, Casamassimi A, Agrawal S, Mendelsohn J, Bianco AR, Ciardiello F. (1999).Oncol Rep. Sep-Oct;6(5):1105-9.

Prewett M, Rockwell P, Rockwell RF, Giorgio NA, Mendelsohn J, Scher HI, Goldstein NI. (1996) J Immunother Emphasis Tumor Immunol. Nov;19(6):419-27.

Prigent, S. A., and Lemoine, N. R. (1992). The type 1 (EGFR-related) family of growth factor receptors and their ligands. Prog Growth Factor Res 4, 1-24.

Ramirez, A., Bravo, A., Jorcano, J. L., and Vidal, M. (1994). Sequences 5' of the bovine keratin 5 gene direct tissue-and cell-type-specific expression of a lacZ gene in the adult and during development. Differentiation 58, 53-64.

Redemann, N., Holzmann, B., von Ruden, T., Wagner, E. F., Schlessinger, J., and Ullrich, A. (1992). Anti-oncogenic activity of signalling-defective epidermal growth factor receptor mutants. Mol Cell Biol 12, 491-8.

Reichmann, E. (1994). Oncogenes and epithelial cell transformation. Semin Cancer Biol 5, 157-65.

Roberts, N.J., et al., (1999), BioTechniques 27; 418 - 422

Rodeck, U., Jost, M., Kari, C., Shih, D. T., Lavker, R. M., Ewert, D. L., and Jensen, P. J. (1997a). EGF-R dependent regulation of keratinocyte survival. J Cell Sci 110, 113-21.

Rodeck, U., Jost, M., DuHadaway, J., Kari, C., Jensen, P. J., Risse, B., and Ewert, D. L. (1997b). Regulation of Bcl-xL expression in human keratinocytes by cell- substratum adhesion and the epidermal growth factor receptor. Proc Natl Acad Sci U S A 94, 5067-72.

Roop, D. R., Lowy, D. R., Tambourin, P. E., Strickland, J., Harper, J. R., Balaschak, M., Spangler, E. F., and Yuspa, S. H. (1986). An activated Harvey ras oncogene produces benign tumours on mouse epidermal tissue. Nature 323, 822-824.

Sibilia, M., and Wagner, E. F. (1995). Strain-dependent epithelial defects in mice lacking the EGF receptor. Science 269, 234-8.

Sibilia, M., Steinbach, J. P., Stingl, L., Aguzzi, A., and Wagner, E. F. (1998). A strain-independent postnatal neurodegeneration in mice lacking the EGF receptor. Embo J 17, 719-31.

Schlessinger, J. (1994). SH2/SH3 signaling proteins. Curr Opin Genet Dev 4, 25-30

Stoll, S. W., Benedict, M., Mitra, R., Hiniker, A., Elder, J. T., and Nunez, G. (1998). EGF receptor signaling inhibits keratinocyte apoptosis: evidence for mediation by Bcl-XL. Oncogene 16, 1493-9.

Threadgill, D. W., Dlugosz, A. A., Hansen, L. A., Tennenbaum, T., Lichti, U., Yee, D., LaMantia, C., Mourton, T., Herrup, K., Harris, R. C., and et al. (1995). Targeted disruption of mouse EGF receptor: effect of genetic background on mutant phenotype. Science 269, 230-4.

Todaro, G. J., Lazar, G. K., and Green, H. (1965). The initiation of cell division in a contact-inhibited mammalian cell line. J Cell Physiol 66, 325-33.

Vardy, D. A., Kari, C., Lazarus, G. S., Jensen, P. J., Zilberstein, A., Plowman, G. D., and Rodeck, U. (1995). Induction of autocrine epidermal growth factor receptor ligands in human keratinocytes by insulin/insulin-like growth factor-1. J Cell Physiol 163, 257-65.

Vassar, R., and Fuchs, E. (1991). Transgenic mice provide new insights into the role of TGF-alpha during epidermal development and differentiation. Genes Dev 5, 714-27.

Wang, W., Fisher, E. M., Jia, Q., Dunn, J. M., Porfiri, E., Downward, J., and Egan, S. E. (1995). The Grb2 binding domain of mSos1 is not required for downstream signal transduction. Nat Genet 10, 294-300.

Weiss, F. U., Daub, H., and Ullrich, A. (1997). Novel mechanisms of RTK signal generation. Curr Biol in Gen and Dev 7, 80-86.

**Claims**

1. Epidermal growth factor receptor (EGFR) inhibitors for the preparation of a medicament for the treatment of tumors whose cells proliferate as a result of a deregulated mitogenic signal transduction pathway and require the function of wildtype EGFR as a survival factor and as an inhibitor of differentiation.

2. EGFR inhibitors for the preparation of a medicament for the treatment of tumors whose cells proliferate as a result of the deregulated Ras signal transduction pathway.

3. The use of claim 2 wherein the deregulation of the Ras pathway is the result of an oncogenic Ras mutation.

Fig. 1a

**B**

Fig. 1b

IB: αHA          IB: αSOS1

mSOS1 170kD

K5-SOS-F 155kD

**Fig. 1c-g**

## Fig. 2

**A**

**B**

## Fig. 3a

**Fig. 3b**

**Fig. 4a-h**

+/+ K5-SOS-F                    wa2/- K5-SOS-F

Ki67

Ki67

Tunel

K1

Fig. 4i

**Fig. 4j**

**Fig. 5a**

**B**

**Keratinocytes**                    **Dermal Fibroblasts**

HB-EGF

EGFR

Fig. 5b

EP 1 170 011 A1

Fig. 5c,d,e

Fig. 6a

**Fig. 6b**

Fig. 7

# EP 1 170 011 A1

**European Patent Office**

## PARTIAL EUROPEAN SEARCH REPORT

which under Rule 45 of the European Patent Convention shall be considered, for the purposes of subsequent proceedings, as the European search report

**Application Number**

EP 00 11 4482

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 31048 A (BRIDGES ALEXANDER JAMES ;WARNER LAMBERT CO (US); DRISCOLL DENISE () 2 June 2000 (2000-06-02) <br> * abstract * <br> * page 1, line 5 - line 12 * <br> * page 2, line 6 - line 26 * <br> * page 5, line 31 - page 6, line 3 * <br> * page 7, line 1 - line 25 * <br> * page 8, line 21 - page 10, line 3 * <br> * page 23, line 3 - page 24, line 4 * <br> * claims 1-5,7,8 * <br> --- | 1-3 | A61K31/5377 <br> A61K31/519 <br> A61K31/517 <br> A61P35/00 |
| D,X | WO 97 02266 A (CIBA GEIGY AG ;TRAXLER PETER (CH); BOLD GUIDO (CH); BRILL WOLFGANG) 23 January 1997 (1997-01-23) <br> * abstract * <br> * page 1, paragraph 1 * <br> * page 6, paragraph 3 - paragraph 4 * <br> * page 8, paragraph 4 - page 9, paragraph 1 * <br> * page 9, paragraph 5 * <br> * example 39 * <br> * claim 12 * <br> --- <br> -/-- | 1-3 | **TECHNICAL FIELDS SEARCHED (Int.Cl.7)** <br> A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 20 August 2001 | Cielen, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

| European Patent | INCOMPLETE SEARCH | Application Number |
|---|---|---|
| Office | SHEET C | EP 00 11 4482 |

Reason for the limitation of the search:

Present claims 1-3 relate to a compounds and their therapeutic applications which are actually not well-defined. The use of the definitions "epidermal growth factor receptor inhibitors", "tumors whose cells proliferate as a result of a deregulated mitogenic signal transduction pathway and require the function of wildtype EGFR as a survival factor and as an inhibitor of differentiation", "tumors whose cells proliferate as a result of the deregulated Ras signal transduction pathway", "wherein the deregulation of teh Ras pathway is a result of an oncogenic Ras mutation" in the present context is considered to lead to a lack of clarity within the meaning of Article 84 EPC. The lack of clarity is such as to render a meaningful complete search impossible. Consequently, the search has been restricted to the compounds specifically mentioned in the description (p. 10, line 30 - p. 11, line 3) and the examples 7-8, namely ZD-1839, CP 358774, CI 1033, PD 183805, PKI-166 and BIBX 1382, in relation to the treatment of cancer.

**European Patent Office**

## PARTIAL EUROPEAN SEARCH REPORT

**Application Number**

EP 00 11 4482

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| D,X | WO 96 30347 A (PFIZER ;SCHNUR RODNEY C (US); ARNOLD LEE D (US)) 3 October 1996 (1996-10-03) * abstract * * page 1, line 7 - line 32 * * page 2, line 12 - line 22 * * page 5, line 22 * * page 19, line 13 - line 23 * * example 20 * * claims 10,19-21 * * page 56, line 13 * --- | 1-3 | |
| D,X | WO 96 33980 A (ZENECA LTD ;GIBSON KEITH HOPKINSON (GB)) 31 October 1996 (1996-10-31) * abstract * * page 1, paragraph 1 * * page 2, paragraph 1 - paragraph 2 * * page 4, paragraph 2 - paragraph 3 * * page 12, paragraph 7 - page 13, paragraph 2 * * page 15, paragraph 7 - paragraph 8 * * page 20, paragraph 2 * * example 10 * * example 32 * * claims 14,15,18 * --- | 1-3 | **TECHNICAL FIELDS SEARCHED** (Int.Cl.7) |
| X | US 5 821 240 A (VON RUEDEN THOMAS ET AL) 13 October 1998 (1998-10-13) * abstract * * column 13, line 51 - line 53 * * column 18, line 56 - column 19, line 4 * * column 20, line 46 - line 52 * * column 21, line 4 - line 12 * * column 44, line 43 - line 44 * --- -/-- | 1-3 | |

EPO FORM 1503 03.82 (P04C10)

EP 1 170 011 A1

**European Patent**
**Office**

**PARTIAL EUROPEAN SEARCH REPORT**

Application Number

EP 00 11 4482

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | EP 0 359 282 A (RORER INT OVERSEAS) 21 March 1990 (1990-03-21) * abstract * * column 1, line 40 - line 50 * * column 3, line 57 - column 4, line 11 * * column 5, line 10 - line 18 * * column 15, line 45 - column 16, line 45 * * claims 1,6,7 * | 1 | |
| X | US 6 084 095 A (DENNY WILLIAM ALEXANDER ET AL) 4 July 2000 (2000-07-04) * abstract * * column 1, line 15 - line 19 * * column 1, line 49 - line 58 * * column 21, line 59 - column 22, line 22 * | 1 | |
| X | HAMILTON MARK ET AL: "Oncogenic Ha-Ras-dependent mitogen-activated protein kinase activity requires signaling through the epidermal growth factor receptor." JOURNAL OF BIOLOGICAL CHEMISTRY, vol. 273, no. 43, 23 October 1998 (1998-10-23), pages 28155-28162, XP001009788 ISSN: 0021-9258 * abstract * * page 28155, column 2, paragraph 1 * * page 28160, column 2, paragraph 2 - page 28161, column 2, paragraph 1 * * figure 6 * | 1-3 | TECHNICAL FIELDS SEARCHED (Int.Cl.7) |

-/--

EPO FORM 1503 03.82 (P04C10)

35

**European Patent Office**

**PARTIAL EUROPEAN SEARCH REPORT**

Application Number

EP 00 11 4482

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | CIARDIELLO F ET AL: "Antitumor effect and potentiation of cytotoxic drugs activity in human cancer cells by ZD - 1839 ( Iressa ), an epidermal growth factor receptor-selective tyrosine kinase inhibitor." CLINICAL CANCER RESEARCH, (2000 MAY) 6 (5) 2053-63., XP001009786 * abstract * * page 2053, column 2, paragraph 1 - page 2054, column 1, paragraph 2 * * figures 1,2 * * page 2055, column 2, paragraph 1 * * page 2059, column 1, paragraph 1 - page 2061, column 1, paragraph 1 * * page 2061, column 2, paragraph 4 - page 2062, column 1, paragraph 1 * | 1-3 | |
| X | BRUNS CHRISTIANE J ET AL: "Blockade of the epidermal growth factor receptor signaling by a novel tyrosine kinase inhibitor leads to apoptosis of endothelial cells and therapy of human pancreatic carcinoma." CANCER RESEARCH, vol. 60, no. 11, 1 June 2000 (2000-06-01), pages 2926-2935, XP001009785 ISSN: 0008-5472 * abstract * * page 2926, column 1, paragraph 2 - column 2, paragraph 4 * * page 2931, column 1, paragraph 2 - column 2, paragraph 1 * * page 2932, column 1, paragraph 2 - column 2, paragraph 1 * * page 2933, column 1, paragraph 2 - column 2, paragraph 1 * | 1-3 | TECHNICAL FIELDS SEARCHED (Int.Cl.7) |

—/—

EPO FORM 1503 03.82 (P04C10)

EPO FORM 1503 03.82 (P04C10)

| | PARTIAL EUROPEAN SEARCH REPORT | Application Number |
|---|---|---|
| European Patent Office | | EP 00 11 4482 |

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | MOYER JAMES D ET AL: "Induction of apoptosis and cell cycle arrest by CP-358,774, an inhibitor of epidermal growth factor receptor tyrosine kinase." CANCER RESEARCH, vol. 57, no. 21, 1 November 1997 (1997-11-01), pages 4838-4848, XP001009787 ISSN: 0008-5472 * abstract * * page 4838, column 1, paragraph 2 - column 2, paragraph 1 * * page 4846, column 1, paragraphs 2,4 * * page 4846, column 2, paragraph 3 - paragraph 4 * * page 4847, column 1, paragraph 2 * | 1-3 | |
| X | KELLY HELEN C ET AL: "ZD1839 ('IRESSA'), an oral epidermal growth factor receptor tyrosine kinase inhibitor (EGFR-TKI): Pharmacokinetics in a phase I study of patients with advanced cancer." PROCEEDINGS OF THE AMERICAN ASSOCIATION FOR CANCER RESEARCH ANNUAL, no. 41, March 2000 (2000-03), pages 612-613, XP001011263 91st Annual Meeting of the American Association for Cancer Research.;San Francisco, California, USA; April 01-05, 2000, March, 2000 ISSN: 0197-016X * the whole document * | 1 | TECHNICAL FIELDS SEARCHED (Int.Cl.7) |
| | -/-- | | |

## PARTIAL EUROPEAN SEARCH REPORT

European Patent Office

Application Number

EP 00 11 4482

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | NUIJEN B ET AL: "In vitro biocompatibility studies with the experimental anticancer agent BIBX1382BS." INTERNATIONAL JOURNAL OF PHARMACEUTICS (AMSTERDAM), vol. 194, no. 2, 25 January 2000 (2000-01-25), pages 261-267, XP001010826 ISSN: 0378-5173 * abstract * * page 261, column 1, paragraph 1 - column 2, paragraph 1 * * figure 1 * | 1 | |
| X | KELLOFF G J ET AL: "EPIDERMAL GROWTH FACTOR RECEPTOR TYROSINE KINASE INHIBITORS AS POTENTIAL CANCER CHEMOPREVENTIVES" CANCER EPIDEMIOLOGY, BIOMARKERS AND PREVENTION,AMERICAN ASSOCIATION FOR CANCER RESEARCH,,US, vol. 5, no. 8, August 1996 (1996-08), pages 657-666, XP000980248 ISSN: 1055-9965 * abstract * * page 658, column 1, paragraph 2 * * page 658, column 2, paragraph 2 - paragraph 3 * * page 661, column 1, paragraph 2 * * page 661, column 1, paragraph 4 - page 663, column 1, paragraph 3 * * page 663, column 2, paragraph 5 - page 664, column 1, paragraph 2 * | 1 | TECHNICAL FIELDS SEARCHED (Int.Cl.7) |

-/--

EPO FORM 1503 03.82 (P04C10)

# EP 1 170 011 A1

European Patent Office

**PARTIAL EUROPEAN SEARCH REPORT**

Application Number

EP 00 11 4482

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | KATO K ET AL: "Oncogenic Ras modulates epidermal growth factor responsiveness in endometrial carcinomas." EUROPEAN JOURNAL OF CANCER, (1998 APR) 34 (5) 737-44., XP001010820 * abstract * * page 737, column 1, paragraph 1 * * page 738, column 1, paragraph 2 - paragraph 3 * * page 742, column 2, paragraph 1 - paragraph 2 * * page 743, column 1, paragraph 2 - column 2, paragraph 1 * | | |
| A | WO 97 40006 A (MARRIOTT JONATHAN ;JARMAN MICHAEL (GB); NEIDLE STEPHEN (GB); CANCE) 30 October 1997 (1997-10-30) * page 1, line 7 - line 9 * * page 6, line 5 - line 21 * | | |
| E | WO 00 47574 A (YANG BINGWEI VERA ;PFIZER PROD INC (US)) 17 August 2000 (2000-08-17) * page 1, line 16 - line 25 * * page 11, line 19 - line 36 * * page 12, line 19 - line 24 * * page 14, line 8 - line 23 * * page 48, line 20 - line 32 * * claims 20,24,25 * | 1-3 | |

TECHNICAL FIELDS SEARCHED (Int.Cl.7)

EPO FORM 1503 03.82 (P04C10)

39

## ANNEX TO THE EUROPEAN SEARCH REPORT
## ON EUROPEAN PATENT APPLICATION NO.

EP 00 11 4482

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2001

| Patent document cited in search report | | Publication date | Patent family member(s) | | Publication date |
|---|---|---|---|---|---|
| WO 0031048 | A | 02-06-2000 | AU | 6261299 A | 13-06-2000 |
| WO 9702266 | A | 23-01-1997 | AU | 707626 B | 15-07-1999 |
| | | | AU | 6414896 A | 05-02-1997 |
| | | | BR | 9609617 A | 25-05-1999 |
| | | | CA | 2224435 A | 23-01-1997 |
| | | | CZ | 9800015 A | 15-04-1998 |
| | | | EA | 980116 A | 29-10-1998 |
| | | | EP | 0836605 A | 22-04-1998 |
| | | | HU | 9900330 A | 28-05-1999 |
| | | | JP | 11508570 T | 27-07-1999 |
| | | | NO | 975956 A | 10-02-1998 |
| | | | NZ | 312665 A | 30-08-1999 |
| | | | PL | 324285 A | 11-05-1998 |
| | | | SI | 9620103 A | 31-10-1998 |
| | | | SK | 398 A | 08-07-1998 |
| | | | TR | 9800012 T | 21-04-1998 |
| | | | US | 6140332 A | 31-10-2000 |
| | | | ZA | 9605723 A | 06-01-1997 |
| WO 9630347 | A | 03-10-1996 | AP | 735 A | 25-02-1999 |
| | | | AU | 3585499 A | 19-08-1999 |
| | | | AU | 703638 B | 25-03-1999 |
| | | | AU | 5040696 A | 10-10-1996 |
| | | | BR | 9601200 A | 06-01-1998 |
| | | | CA | 2216796 A | 03-10-1996 |
| | | | CN | 1137037 A,B | 04-12-1996 |
| | | | CZ | 9600931 A | 12-02-1997 |
| | | | EP | 1110953 A | 27-06-2001 |
| | | | EP | 0817775 A | 14-01-1998 |
| | | | FI | 973832 A | 29-09-1997 |
| | | | HR | 960147 A | 31-08-1997 |
| | | | HU | 9600834 A | 28-05-1997 |
| | | | JP | 10506633 T | 30-06-1998 |
| | | | JP | 3088018 B | 18-09-2000 |
| | | | KR | 232335 B | 01-12-1999 |
| | | | NO | 961299 A | 01-10-1996 |
| | | | NZ | 286263 A | 24-11-1997 |
| | | | PL | 313541 A | 14-10-1996 |
| | | | SG | 43262 A | 17-10-1997 |
| | | | SI | 9600102 A | 28-02-1997 |
| | | | SK | 38796 A | 06-08-1997 |
| | | | TR | 970153 A | 21-03-1997 |
| WO 9633980 | A | 31-10-1996 | AT | 198329 T | 15-01-2001 |
| | | | AU | 699163 B | 26-11-1998 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 00 11 4482

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2001

| Patent document cited in search report | Publication date | Patent family member(s) | | Publication date |
|---|---|---|---|---|
| WO 9633980 A | | AU 5343396 A | | 18-11-1996 |
| | | BG 62730 B | | 30-06-2000 |
| | | BG 102052 A | | 31-08-1998 |
| | | BR 9608082 A | | 26-01-1999 |
| | | CA 2215732 A | | 31-10-1996 |
| | | CN 1182421 A | | 20-05-1998 |
| | | CZ 9703396 A | | 18-02-1998 |
| | | DE 69611361 D | | 01-02-2001 |
| | | DE 69611361 T | | 26-04-2001 |
| | | DK 823900 T | | 02-04-2001 |
| | | EE 9700252 A | | 15-04-1998 |
| | | EP 0823900 A | | 18-02-1998 |
| | | ES 2153098 T | | 16-02-2001 |
| | | HR 960204 A | | 31-08-1997 |
| | | HU 9802839 A | | 29-03-1999 |
| | | JP 3040486 B | | 15-05-2000 |
| | | JP 11504033 T | | 06-04-1999 |
| | | NO 974940 A | | 24-10-1997 |
| | | NZ 305444 A | | 29-03-1999 |
| | | PL 323066 A | | 02-03-1998 |
| | | PT 823900 T | | 30-04-2001 |
| | | SI 823900 T | | 30-06-2001 |
| | | SK 145497 A | | 04-02-1998 |
| | | US 5770599 A | | 23-06-1998 |
| | | ZA 9603358 A | | 28-10-1996 |
| US 5821240 A | 13-10-1998 | DE 19608588 A | | 11-09-1997 |
| | | AU 730376 B | | 08-03-2001 |
| | | AU 2094597 A | | 22-09-1997 |
| | | BG 63163 B | | 31-05-2001 |
| | | BG 102789 A | | 30-09-1999 |
| | | BR 9707839 A | | 27-07-1999 |
| | | CA 2248720 A | | 12-09-1997 |
| | | CN 1212694 A,B | | 31-03-1999 |
| | | CZ 9802815 A | | 17-02-1999 |
| | | EE 9800278 A | | 15-02-1999 |
| | | WO 9732880 A | | 12-09-1997 |
| | | EP 0888351 A | | 07-01-1999 |
| | | HU 9902049 A | | 28-10-1999 |
| | | JP 2000506151 T | | 23-05-2000 |
| | | NO 984082 A | | 04-09-1998 |
| | | PL 328719 A | | 15-02-1999 |
| | | SK 120898 A | | 11-02-1999 |
| | | TR 9801755 T | | 21-12-1998 |
| | | ZA 9701887 A | | 07-09-1998 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.** EP 00 11 4482

This annex lists the patent family membersrelating to the patent documents cited in the above–mentioned European search report.
The members are as contained in the European Patent Office EDP file on
The European Patent Office is in no way liable for these particulars which are merely given for the purpose of information.

20-08-2001

| Patent document cited in search report | | Publication date | Patent family member(s) | | Publication date |
|---|---|---|---|---|---|
| EP 0359282 | A | 21-03-1990 | AT | 123070 T | 15-06-1995 |
| | | | AU | 4128089 A | 22-03-1990 |
| | | | CA | 1340417 A | 02-03-1999 |
| | | | DE | 68922808 D | 29-06-1995 |
| | | | DE | 68922808 T | 21-09-1995 |
| | | | EP | 0667165 A | 16-08-1995 |
| | | | ES | 2075019 T | 01-10-1995 |
| | | | JP | 2291295 A | 03-12-1990 |
| | | | US | 6217866 B | 17-04-2001 |
| US 6084095 | A | 04-07-2000 | US | 5654307 A | 05-08-1997 |
| | | | US | 6265410 B | 24-07-2001 |
| | | | AU | 686334 B | 05-02-1998 |
| | | | AU | 1731495 A | 08-08-1995 |
| | | | BG | 100614 A | 31-03-1997 |
| | | | CA | 2177372 A | 27-07-1995 |
| | | | CZ | 9601970 A | 17-09-1997 |
| | | | EP | 0742717 A | 20-11-1996 |
| | | | FI | 962856 A | 25-09-1996 |
| | | | HR | 950034 A | 31-10-1997 |
| | | | JP | 9508127 T | 19-08-1997 |
| | | | MD | 960217 A | 30-04-1998 |
| | | | NO | 963094 A | 24-07-1996 |
| | | | PL | 315633 A | 25-11-1996 |
| | | | SK | 89496 A | 08-10-1997 |
| | | | WO | 9519774 A | 27-07-1995 |
| | | | AU | 686339 B | 05-02-1998 |
| | | | AU | 1833495 A | 08-08-1995 |
| | | | BG | 100615 A | 28-02-1997 |
| | | | CA | 2177392 A | 27-07-1995 |
| | | | CN | 1139383 A | 01-01-1997 |
| | | | CN | 1139430 A | 01-01-1997 |
| | | | CZ | 9601971 A | 16-07-1997 |
| | | | EP | 0741711 A | 13-11-1996 |
| | | | FI | 962855 A | 13-09-1996 |
| | | | HR | 950033 A | 31-10-1997 |
| | | | HU | 74590 A | 28-01-1997 |
| | | | HU | 74589 A | 28-01-1997 |
| | | | JP | 9508126 T | 19-08-1997 |
| | | | MD | 960211 A | 30-04-1998 |
| | | | NO | 963093 A | 24-07-1996 |
| | | | NZ | 281404 A | 28-05-1999 |
| | | | PL | 315632 A | 25-11-1996 |
| | | | SK | 89596 A | 06-08-1997 |
| | | | WO | 9519970 A | 27-07-1995 |
| | | | US | 5679683 A | 21-10-1997 |

**ANNEX TO THE EUROPEAN SEARCH REPORT
ON EUROPEAN PATENT APPLICATION NO.**

EP 00 11 4482

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2001

| Patent document cited in search report | | Publication date | Patent family member(s) | | Publication date |
|---|---|---|---|---|---|
| US 6084095 | A | | ZA 9500440 A | | 10-10-1995 |
| | | | ZA 9500441 A | | 10-10-1995 |
| WO 9740006 | A | 30-10-1997 | AU 2644597 A | | 12-11-1997 |
| | | | EP 0902779 A | | 24-03-1999 |
| | | | JP 2000509034 T | | 18-07-2000 |
| WO 0047574 | A | 17-08-2000 | AU 2124800 A | | 29-08-2000 |
| | | | US 6258824 B | | 10-07-2001 |

EPO FORM P0459

For more details about this annex : see Official Journal of the European Patent Office, No. 12/82